(19) Europäisches Patentamt
European Patent Office
Office européen des brevets

(11) **EP 4 137 795 A1**

(12) **EUROPEAN PATENT APPLICATION**

(43) Date of publication:
**22.02.2023 Bulletin 2023/08**

(21) Application number: **22190967.4**

(22) Date of filing: **18.08.2022**

(51) International Patent Classification (IPC):
**G01N 15/14** (2006.01)    **G01N 21/64** (2006.01)
**G06T 7/00** (2006.01)    **G01N 15/10** (2006.01)

(52) Cooperative Patent Classification (CPC):
**G01N 15/1475; G01N 15/147; G01N 21/6428;**
**G01N 21/6458; G06T 7/0012;** G01N 2015/1006;
G01N 2015/1472; G01N 2021/6441;
G06T 2207/10024; G06T 2207/10056;
G06T 2207/10064; G06T 2207/30024

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **18.08.2021 JP 2021133166**

(71) Applicants:
• **National University Corporation Tokyo Medical and Dental University**
**Tokyo 113-8510 (JP)**
• **Kyoto Prefectural Public University Corporation Kyoto-shi, Kyoto 602-8566 (JP)**

• **SYSMEX CORPORATION**
**Kobe-shi**
**Hyogo 651-0073 (JP)**

(72) Inventors:
• **Inazawa, Johji**
**Tokyo, 113-8510 (JP)**
• **Kuroda, Junya**
**Kyoto-shi, Kyoto, 602-8566 (JP)**
• **Kinoshita, Masaki**
**Kobe-shi, Hyogo, 651-0073 (JP)**

(74) Representative: **Hoffmann Eitle**
**Patent- und Rechtsanwälte PartmbB**
**Arabellastraße 30**
**81925 München (DE)**

(54) **FLUORESCENCE IMAGE ANALYSIS METHOD, FLUORESCENCE IMAGE ANALYSER, FLUORESENCE IMAGE ANALYSIS PROGRAM**

(57) A fluorescence image analyzer 10 has an imaging unit 154 for capturing a first image containing at least a part of a region of a cell as an imaging target for a plurality of cells in a sample in which a target site on a chromosome is labeled with a fluorescent dye, and a second image including fluorescence generated from a fluorescent dye labeling the target site of the cell of the first image. The processing unit 11 of the fluorescence image analyzer 10 selects a plurality of test cells having specific morphological characteristics to be tested from a plurality of cells based on at least the first image, and extracts the bright spots of fluorescence generated from the fluorescent dye from at least a plurality of test cells. The processing unit 11 identifies cells with chromosomal abnormalities and/or cells without chromosomal abnormalities based on the extracted bright spots, and generates information related to the ratio of cells with chromosomal abnormalities relative to the test cells based on the number of cells with chromosomal abnormalities and/or the number of cells without chromosomal abnormalities.

FIG. 1

EP 4 137 795 A1

**Description**

CROSS REFERENCE TO RELATED APPLICATIONS

This application claims priority from prior Japanese Patent Application(s) No. 2021-133166, filed on August 18, 2021, entitled "FLUORESCENCE IMAGE ANALYSIS METHOD, FLUORESCENCE IMAGE ANALYZER, FLUORESCENCE IMAGE ANALYSIS PROGRAM", the entire contents of which are incorporated herein by reference.

FIELD OF THE INVENTION

The present invention relates to a fluorescence image analysis method, fluorescence image analyzer, and fluorescence image analysis program

BACKGROUND

Conventionally, a method using a fluorescence in situ hybridization (FISH method) has been used as a method for detecting positive cells having chromosomal abnormalities such as chromosomal translocation, chromosomal inversion, gene deletion, or gene amplification. According to the FISH method, cells are labeled by pretreatment in which a labeled probe is hybridized to the DNA sequence region to be detected in the cell, and the examiner observes the fluorescence generated by the labeled probe with a fluorescence microscope and samples to determine if there are positive cells. In the diagnosis of the subject, depending on the disease, if the ratio of positive cells to the examined cells is 1% to several%, it is diagnosed that treatment or detailed examination is required. In the above method in which the examiner determines positive cells using a fluorescence microscope, the number of cells determined by the examiner is generally 100 to 200 at most per sample. Therefore, in order not to overlook positive cells, specific types of cells are isolated from biological samples using antibody beads, and the isolated cells are inspected. However, isolating a particular type of cell from a biological sample has been a cumbersome task for the examiner. As a method that does not require such complicated isolation work, Japanese Patent Application Publication No. 2017-215311 discloses a method wherein a two-dimensional fluorescent image obtained by flowing detection cells labeled with a labeled probe into a flow cell and imaging the detection cells flowing through the flow cell, and the bright spots are extracted from this image for each detected cell via software, and based on the extracted bright spots, it is determined whether each detected cell is a positive cell, and the ratio of the positive cell to the detected cell is output as a fluorescent image. According to the analysis method disclosed in Japanese Patent Application Publication No. 2017-215311, the number of detected cells per sample is increased by several tens to tens of thousands of times as compared with the above method in which an examiner determines positive cells using a fluorescence microscope. Therefore, it is not necessary to isolate a specific type of cell from a biological sample.

However, according to the analysis method disclosed in Japanese Patent Application Publication No. 2017-215311, for example, depending on the orientation of the cells in the flow cell at the time of imaging, a plurality of bright spots may overlap in the fluorescent image of the negative cells, and there is a risk that the software will include these negative cells as positive cells. Since the probability of such a determination is small, it generally does not affect the diagnosis of the subject, but when the ratio of positive cells to the examined cells is determined to be 1% to several% in the case of diagnosis of a disease requiring treatment or detailed examination, there is a problem that the influence of the above determination becomes large and the diagnosis becomes difficult.

Therefore, the present invention provides a fluorescence image analysis method, a fluorescence image analyzer, and a fluorescence image analysis program that can facilitate the diagnosis of a subject.

SUMMARY OF THE INVENTION

The fluorescence image analysis method of the present invention includes labeling a target site on a chromosome with fluorescent dye in a plurality of cells contained in a sample; capturing, for a plurality of the cells in the sample, a first image containing at least a part of the cell region in the imaging target, and a second image containing the fluorescence generated from the fluorescent dye labeled at the target site of the cell in the first image; selecting a plurality of test cells having specific morphological characteristics to be tested from the plurality of cells based on at least the first image; extracting, bright spots due to fluorescence generated from the fluorescent dye from at least a plurality of the test cells based on the second image; identifying cells with chromosomal abnormalities and/or cells without chromosomal abnormalities based on the extracted bright spots; and generating information related to the ratio of cells with chromosomal abnormality to the test cells based on the number of cells with the chromosomal abnormality and/or the number of cells without chromosomal abnormality.

The fluorescence image analyzer of the present invention includes an imaging unit for capturing a first image including a region of at least a part of the cells in a sample as an imaging target, and capturing a second image including the fluorescence generated from the fluorescent dye labeling the target site of the cells of the first image as an imaging target; and a processing unit; wherein the processing unit executes a process for selecting a plurality of test cells having specific morphological characteristics to be tested from the plurality of cells based on at least the first image; a process for extracting, bright spots due to fluorescence generated from the fluorescent dye from at least a plurality of the test cells based on the second image; a process for identifying, based on the extracted bright spots, cells with chromosomal abnormalities and/or cells without chromosomal abnormalities, and a process for generating information related to the ratio of cells having the chromosomal abnormality to the test cells based on the number of cells with the chromosomal abnormality and/or the number of cells without the chromosomal abnormality.

The fluorescence image analysis computer-executable program of the present invention executes a process for obtaining a first image including at least part of the cells to be imaged, and a second image including fluorescence generated from the fluorescent dye labeling the target site of the cells of the first image as an imaging target for a plurality of cells in the sample having a target site on the chromosome labeled with a fluorescent dye; a process for selecting a plurality of test cells having specific morphological characteristics to be tested from the plurality of cells based on at least the first image; a process for extracting, bright spots due to fluorescence generated from the fluorescent dye from at least a plurality of the test cells based on the second image; a process for identifying cells having a chromosomal abnormality and/or cells having no chromosomal abnormality based on the extracted bright spots; and a process for generating information related to the ratio of cells with chromosomal abnormality to the test cells based on the number of cells with the chromosomal abnormality and/or the number of cells without chromosomal abnormality.

Therefore, the present invention provides a fluorescence image analysis method, a fluorescence image analyzer, and a fluorescence image analysis program that can facilitate the diagnosis of a subject.

BRIEF DESCRIPTION OF THE DRAWINGS

FIG. 1 is a diagram schematically showing the structure of a fluorescence image analyzer which is an example of an embodiment;

FIG. 2 is a diagram showing examples of a first image, two second images, a third image, and a composite image of two second images acquired by a fluorescence image analyzer;

FIG. 3 is a schematic composite image of a first image and a third image, and is a diagram illustrating a method of calculating a nuclear uneven distribution value by a processing unit;

FIG. 4 shows a graph plotting the nuclear uneven distribution value and cell area of each cell contained in a sample prepared from the bone marrow fluid of one sample in two-dimensional coordinates in which the nuclear uneven distribution value is a parameter on the horizontal axis and the cell area is a parameter on the vertical axis;

FIG. 5A is a diagram showing a nuclear image (a), a bright-field image (b), and a composite image (c) thereof in a general multiple myeloma cell;

FIG. 5B shows a nuclear image (a), a bright-field image (b), and a composite image (c) thereof in non-multiple myeloma cells;

FIG. 6A schematically shows a third image, FIG. 6B shows a first second image, and FIG. 6C shows second second image (c) taken from the same region of a sample flowing through a flow cell;

FIG. 7 is a diagram illustrating cell classification by a processing unit based on a bright spot pattern, and is a schematic diagram showing a simplified bright spot pattern;

FIG. 8 is a diagram for showing a method of bright spot extraction/fusion determination by a processing unit;

FIG. 9 is a diagram for describing a method of fusion determination by a processing unit;

FIG. 10 is a flowchart showing an example of a procedure of fluorescence image analysis processing executed in the processing unit;

FIG. 11 is a flowchart showing an example of a detailed procedure of the classification process of step S2 of FIG. 10;

FIG. 12 is a flowchart showing an example of a detailed procedure of the classification process of step S3 of FIG. 10;

FIG. 13 is a graph showing the calculation results when the positive cell rates were calculated for the IGH/CCND1 fusion gene, the IGH/FGFR3 fusion gene, and the IGH/MAF fusion gene in the bone marrow specimen of a multiple myeloma patient having an IGH translocation;

FIG. 14 is a graph showing the calculation results when the positive cell rates were calculated for each of the IGH/CCND1 fusion gene, the IGH/FGFR3 fusion gene, and the IGH/MAF fusion gene in a bone marrow sample of a patient with multiple myeloma who did not have an IGH translocation;

FIG. 15 is a graph plotting the nuclear area and cell area in each cell contained in a sample prepared from a spleen tissue sample of a follicular lymphoma patient having an IGH translocation, in two-dimensional coordinates with the nuclear area as a parameter on the horizontal axis and the cell area as a parameter on the vertical axis;

FIG. 16 is a graph showing the calculation results when the positive cell rates of the IGH/CCND1 fusion gene, the IGH/BCL2 fusion gene, and the IGH/MYC fusion gene are calculated for each sample of the follicular lymphoma patient;

FIG. 17 is a graph plotting the nuclear area and cell area of each cell contained in a sample prepared from a lymph node sample of a patient with diffuse large cell type B-cell lymphoma having an IGH translocation in two-dimensional coordinates with the nuclear area as a parameter on the horizontal axis, and the cell area as a parameter on the vertical axis;

FIG. 18 is a graph showing the calculation results when the positive cell rates of the IGH/CCND1 fusion gene, the IGH/BCL2 fusion gene, and the IGH/MYC fusion gene are calculated for each sample of the diffuse large cell type B-cell lymphoma patient;

FIG. 19 is a flowchart showing a modified example of the classification process of step S3 of FIG. 10;

FIG. 20 is a flowchart showing a modified example of the classification process of step S3 of FIG. 10;

FIG. 21 is a schematic view of a fluorescence image analyzer which is another example of an embodiment; and

FIG. 22 is a schematic diagram of a fluorescence image analyzer which is another example of an embodiment.

DESCRIPTION OF THE EMBODIMENTS OF THE INVENTION

Hereinafter, embodiments of the fluorescence image display method and the fluorescence image analyzer according to the present invention will be described in detail with reference to the drawings. The embodiments described below are merely examples, and the present invention is not limited to the following embodiments. Among the components described below, the components not described in the independent claims indicating the highest level concept are arbitrary components and are not essential components. Note that the present invention is not limited to the embodiments and modifications described below, and the design can be appropriately changed as long as the object of the present invention is not impaired. For example, it is within the scope of the present disclosure to selectively combine the components of the plurality of embodiments and modifications described below.

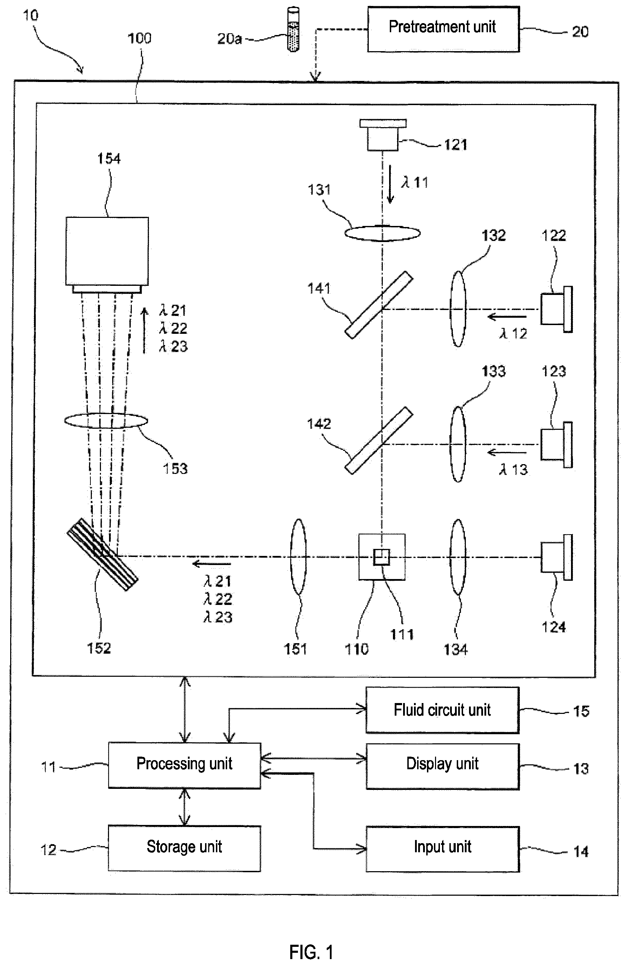

FIG. 1 is a diagram schematically showing a configuration of a fluorescence image analyzer 10 which is an example of an embodiment. In FIG. 1, the solid double-headed arrow indicates a signal line, the alternate long and short-dashed line indicates the light path, and the solid single-headed arrow indicates the traveling direction of light. As shown in FIG. 1, the fluorescence image analyzer 10 includes an imaging unit 100 that acquires a fluorescent image and a bright-field image of cells with target sites that are labeled with a fluorescent dye (hereinafter, may be referred to as "fluorescent-labeled cells") for each cell, a fluid circuit unit 15, and a processing unit 11 that controls each device such as the imaging unit 100 and the fluid circuit unit 15 and generates information on the ratio of cells in which gene fusion due to chromosomal translocation occurs. The fluorescence image analyzer 10 also includes a storage unit 12, a display unit 13, and an input unit 14 connected to the processing unit 11.

As will be described in detail later, in this embodiment, image analysis by software is performed based on a bright-field image as an example of a first image, two fluorescent bright spot images as an example of a second image, and a nuclear-stained image as an example of a third image. Then, the plurality of cells contained in the sample are classified into a plurality of test cells having a specific morphological characteristic and a plurality of non-test cells not having the specific morphological characteristic, and further, a plurality of test cells classified as indeterminate cells, negative cells, and positive cells. As used herein, a positive cell means a cell having a chromosomal abnormality, and a negative cell means a cell having no chromosomal abnormality. Chromosomal abnormalities include chromosomal translocations, chromosomal inversions, gene deletions, or gene amplification.

The imaging unit 100 includes light sources 121 to 124 that irradiate light on fluorescently labeled cells, a flow cell 110 for flowing a sample 20a, and an image capture unit 154. The imaging unit 100 includes condenser lenses 131 to 134, 151, 153, a dichroic mirror 141, 142, and an optical unit 152. In the present embodiment, the imaging unit 100 is provided with four types of light sources 121 to 124 having different wavelengths of output light. The image capture unit 154 captures three types of fluorescent images using three types of fluorescent dyes having different wavelengths of excitation light and output fluorescence, and a bright-field image of cells. Note that the bright-field image of a cell is an image obtained by uniformly irradiating the entire cell with light and detecting the light transmitted through the cell.

In the present embodiment, the sample 20a was a bone marrow fluid prepared by pretreatment including a step of hybridizing two different types of nucleic acid probes labeled with the first fluorescent dye and the second fluorescent dye with the target site in the nucleic acid, and a nuclear staining step of labeling the nucleus of each cell with a dye for nuclear staining as a third fluorescent dye. Then, using the fluorescence image analyzer 10, information on the ratio of positive cells based on the number of negative cells and the number of positive cells is calculated from a plurality of test cells selected from the plurality of cells contained in the sample 20a.

Note that, in the present embodiment, the case wherein the positive cell is a multiple myeloma cell (hereinafter, simply referred to as myeloid seed cell) in which the IGH gene or the FGFR3 gene is fused by a chromosomal translocation to generate the IGH/FGFR3 fusion gene, and the negative cell is a cell containing no IGH/FGFR3 fusion gene will be described. As will be described later in a modified example, the fluorescence image display method and apparatus according to the present invention are not limited to myeloma cells containing the IGH/FGFR3 fusion gene, insofar as the measurement target also may be myeloma cells including other fusion genes. Alternatively, the measurement target may be cells other than plasma cells (bone marrow seed cells), and may be nucleated cells. Note that in the following description, the IGH/FGFR3 fusion gene also may be referred to as t (4; 14). Here, in the present specification, "t" means a translocation, and "x" and "y" when expressed as t (x; y) mean a chromosome number in which each gene is present.

The fluorescence image analyzer 10 measures the sample 20a prepared by the pretreatment unit 20 and classifies the cells contained in the sample 20a. In the pretreatment unit 20, a sample 20a is prepared by performing a pretreatment including a step of labeling the target site of the cell with a fluorescent dye and a step of specifically staining the nucleus of the cell with a dye for nuclear staining. In the step of labeling the target site with the fluorescent dye, the nucleic acid probe labeled with the fluorescent dye and the target site in the nucleic acid are hybridized.

The first nucleic acid probe that hybridizes with the IGH gene is labeled with a first fluorescent dye that emits a first fluorescence of wavelength $\lambda 21$ when irradiated with excitation light of wavelength $\lambda 11$. By hybridizing the first nucleic acid probe and the IGH gene, the IGH gene is labeled with the first fluorescent dye. The second nucleic acid probe that hybridizes with the FGFR3 gene is labeled with a second fluorescent dye that emits a second fluorescence at wavelength $\lambda 22$ when irradiated with excitation light of wavelength $\lambda 12$. By hybridizing the second nucleic acid probe with the FGFR3 gene, the FGFR3 gene is labeled with the second fluorescent dye. The nucleus is stained with a dye for nuclear staining that emits a third fluorescence of wavelength $\lambda 23$ when irradiated with excitation light of wavelength $\lambda 13$. Note that whereas Texas Red (registered trademark) is used as the first fluorescent dye, FITC (Fluorescein isothiocyanate) is used as the second fluorescent dye, and Hoechst (registered trademark) 33342 is used as the dye for nuclear staining, the invention is not limited to the use of these dyes.

The flow cell 110 is made of a translucent resin or glass and has a flow path 111 for flowing the sample 20a. The flow cell 110 is provided in a common optical path of the light sources 121 to 124. In the imaging unit 100, the light sources 121 to 124 irradiate light on the flow cell 110, and the image capture unit 154 is configured to take a fluorescence image and a bright-field image of fluorescently labeled cells flowing through the flow path 111 of the flow cell 110.

According to the FISH method using the flow cell 110 (hereinafter referred to as "flow FISH method"), a sample 20a containing fluorescently labeled cells is flowed through the flow cell 110, and a fluorescence image is acquired by imaging the cells in the fluid. In this way the number of cells to be analyzed can be increased on the order of tens to tens of thousands of times as compared with the conventional FISH method by microscopic observation, and high inspection accuracy can be obtained. In particular, reproducibility is improved when the number of positive cells is small.

As described above, the imaging unit 100 is configured such that the light emitted from the light sources 121 to 124 irradiates the sample 20a flowing through the flow path 111 of the flow cell 110. An example of the light sources 121 to 123 is a semiconductor laser light source, and an example of the light source 124 is a white LED. The light source 121 is a light source for exciting the first fluorescent dye, and emits a laser beam having a wavelength of $\lambda 11$. The light source 122 is a light source for exciting the second fluorescent dye, and emits a laser beam having a wavelength of $\lambda 12$. The light source 123 is a light source for exciting a dye for nuclear dyeing as a third fluorescent dye, and emits a laser beam having a wavelength of $\lambda 13$. The light source 124 emits light for generating a bright-field image of the cell, that is, white light transmitted through the cell. In this embodiment, the wavelength $\lambda 11$ is 592 nm, the wavelength $\lambda 12$ is 488 nm, and the wavelength $\lambda 13$ is 405 nm.

The condenser lenses 131 to 134 are arranged between the light sources 121 to 124 and the flow cell 110, respectively, and collect the light emitted from the light sources 121 to 124 on the flow cell 110. The dichroic mirror 141 transmits light of wavelength $\lambda 11$ and reflects light of wavelength $\lambda 12$. The dichroic mirror 142 transmits light having wavelengths $\lambda 11$ and $\lambda 12$ and reflects light having wavelengths $\lambda 13$. By providing such an optical system, the light from the light sources 121 to 124 is applied to the flow path 111 of the flow cell 110. Then, when the sample 20a flowing through the flow path 111 is irradiated with light having wavelengths $\lambda 11$ to $\lambda 13$, the fluorescent dye labeling the cells emits fluorescence.

Specifically, when light having a wavelength of $\lambda 11$ is applied to the first fluorescent dye that labels the IGH gene, light of wavelength of $\lambda 21$ is produced from the first fluorescent dye. When light of wavelength $\lambda 12$ is applied to the second fluorescent dye that labels the FGFR3 gene, the second fluorescent dye produces a second fluorescence of wavelength $\lambda 22$. When light having a wavelength of $\lambda 13$ is applied to a dye for nuclear staining that stains nuclei, a third fluorescence having a wavelength of $\lambda 23$ is produced from the dye for nuclear staining. In the present embodiment, the first fluorescent color is red, the second fluorescent color is green, and the third fluorescent color is blue. When the sample 20a is irradiated with the white light of the light source 124, a part of this light passes through the cells and a bright-field image is obtained. Note that the wavelength of each light source and each fluorescent dye are not limited to those described above, and may be appropriately selected depending on the gene to be detected.

A condenser lens 151, an optical unit 152, and a condenser lens 153 are arranged in this order between the flow cell 110 and the image capture unit 154 from the flow cell 110 side along the optical path of the laser beam. The condenser lens 151 condenses the first to third fluorescence generated from the sample 20a and the transmitted light transmitted through the sample 20a on the optical unit 152. The optical unit 152 is configured by stacking four dichroic mirrors. The four dichroic mirrors reflect the first to third fluorescences at slightly different angles and separate them on the light receiving surface of the image capture unit 154. The condenser lens 153 collects the light reflected by the optical unit 152 on the light receiving surface of the image capture unit 154.

The image capture unit 154 is configured by a TDI (Time Delay Integration) camera. The image capture unit 154 captures an image formed by the first to third fluorescence and transmitted light, acquires includes three types of fluorescence images corresponding to the first to third fluorescences and a bright-field image corresponding to the transmitted light, and the acquired images are stored in a storage unit 12 described later under the control of the processing unit 11.

The bright-field image is an example of the first image, each of the images obtained by capturing the first fluorescence and the second fluorescence is an example of the second image, and the image of the nucleus obtained by capturing the third fluorescence constitutes the third image. Hereinafter, the image obtained by capturing the first fluorescence is referred to as a first second image, and the image obtained by capturing the second fluorescence is referred to as a second second image. The processing unit 11 corrects each image by software so that the positional relationship between the subject and the pixel matches between the images sent from the image capture unit 154. The two second images are preferably the same size as each other in order to analyze the overlap of bright spots.

The TDI camera configuring the image capture unit 154 repeatedly images cells with a plurality of rows of line sensors along the direction of the flow path 111 of the flow cell 110, that is, along the direction in which the sample 20a flows, and obtains and image of the cell by integrating the charge of the line sensor along the direction of the sample flow. In this way a high-quality cell image can be obtained without slowing down the moving speed of the sample 20a and without shortening the exposure time. The configuration of the imaging unit 100 is not limited to the above, and may be appropriately changed as long as the cell image necessary for image analysis by software can be captured. For example, as the optical unit 152, a configuration in which prisms are combined may be adopted. As such an optical unit 152, for example, the optical block described in US Patent Publication No. 2020-0271585 can be used, which is incorporated herein by reference. A CCD camera also may be adopted as the image capture unit 154.

The fluid circuit unit 15 includes a suction pipe for suctioning the sample 20a, a flow path for connecting the suction pipe and the flow cell 110, and a pump; the sample 20a is suctioned by the suction pipe and supplied to the flow cell 110 under the control of the processing unit 11.

The processing unit 11 analyzes the fluorescence image acquired by the imaging unit 100 by executing the software stored in the storage unit 12 described later, and classifies each cell contained in the fluorescence image as negative cells, positive cells, and indeterminate cells, which are fluorescence image cells that do not meet the predetermined conditions for classification into negative cells or positive cells by software. The processing unit 11 is composed of a CPU and executes arithmetic processing related to processing/analysis of a fluorescence image. The processing unit 11 executes various processes including image analysis of the first to third images based on the program stored in the storage unit 12. The processing unit 11 is connected to an imaging unit 100, a storage unit 12, a display unit 13, an input unit 14, and a fluid circuit unit 15, receives signals from each device to acquire various information, outputs control signals to each device to control each device. For example, the processing unit 11 suctions the sample 20a through the suction pipe by controlling the pump of the fluid circuit unit 15, and supplies the suctioned sample 20a to the flow cell 110. Further, the processing unit 11 captures a first image, two second images, and a third image for each cell flowing through the flow cell 110 by controlling the light sources 121 to 124 and the image capture unit 154.

The storage unit 12 is composed of a RAM, a ROM, a solid state drive (SSD), and the like. The storage unit 12 stores software executed by the processing unit 11 for image analysis of the first to third images. The display unit 13 is composed of a liquid crystal display, and displays classification results of each cell, first to third images, information on the ratio of positive cells, and the like. The input unit 14 is composed of a mouse and a keyboard, and is used for inputting information such as a sample ID, switching display screens, and selecting first to third images. Note that the structures of the storage unit 12, the display unit 13, and the input unit 14 are not particularly limited.

The processing unit 11 processes the first to third images captured by the image pickup unit 154 by executing the software stored in the storage unit 12, and the cell size from the first image, fluorescence bright spots related to two types of fluorescence are extracted from the two second images, and the nuclear region is extracted from the third image. Then the processing unit 11 performs analysis based on the extracted cell size, two types of fluorescent bright spots, and a nuclear region, and the numbers of negative cells and the positive cells are calculated from the plurality of cells contained in the sample 20a.

First, the processing unit 11 uses the first image and the third image to compare the target plasma cells and cancerous myeloma cells with the among the plurality of cells of other regions contained in the sample 20a, and selects cells belonging to a region that has frequently contained such cells (hereinafter, cells belonging to this region may be referred to as "test cells"). The region in which the target plasma cells and the cancerous myeloma cells are contained more frequently than in other regions is a region in the distribution map of a plurality of cells contained in the sample 20a, and will be described in detail later. Subsequently, the processing unit 11 analyzes the two second images for the plurality of selected test cells, counts the number of positive cells and the number of negative cells, and calculates information on the ratio of positive cells.

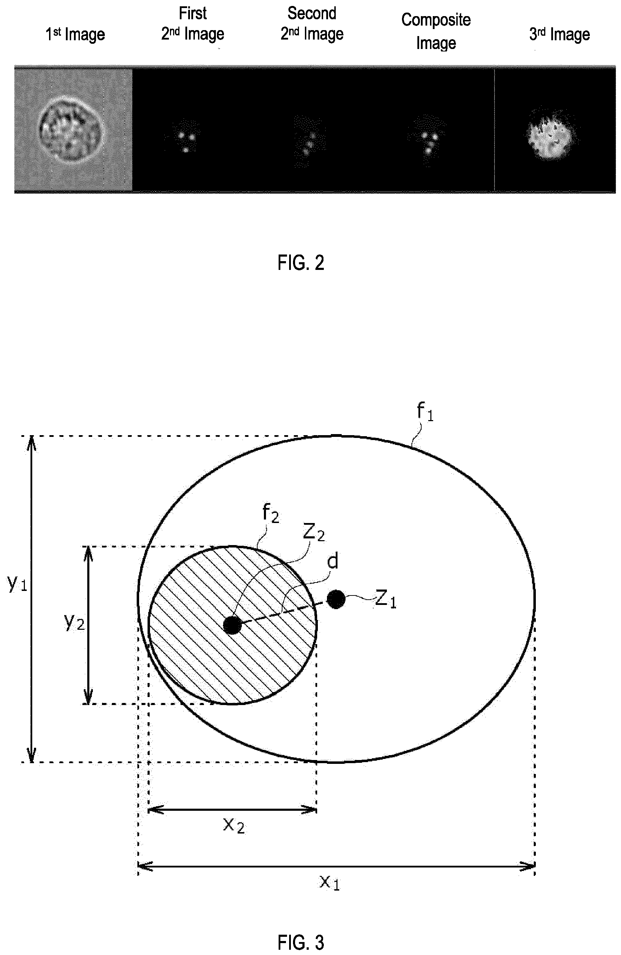

Specifically, the image capture unit 154 captures the first to third images shown in FIG. 2 for each of a plurality of cells (for example, all cells) contained in the sample 20a. The first image, the third image, the two second images, and their composite image shown in FIG. 2 have their color tones changed to gray after the gradation is inverted. First, the processing unit 11 calculates the cell area representing the cell size for each cell contained in the sample 20a based on the first image (bright-field image). The cell area can be calculated by the processing unit 11 counting the number of pixels in which a part of the cell is shown in the bright-field image.

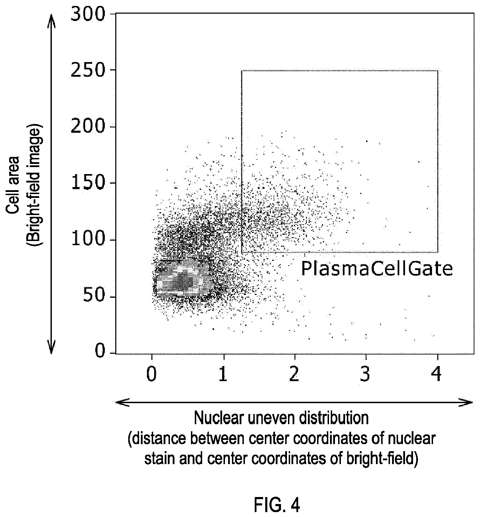

In the present embodiment, the cell size and the nuclear uneven distribution value, which represents the scale of nuclear uneven distribution described below, are used to select a plurality of test cells belonging to a region in which the plasma cells and myeloma cells to be tested are contained more frequently than in other regions from the plurality of cells contained in the sample 20a. The selection of test cells using the cell size and the uneven distribution value of the nucleus will be described later with reference to FIG. 4. The processing unit 11 calculates the nuclear uneven distribution value based on the first image and the third image (nuclear stained image). FIG. 3 is a schematic composite image of the first image and the third image, and is a diagram illustrating a method of calculating a nuclear uneven distribution value in the processing unit 11. In FIG. 3, the circumference of the outer circle f1 indicates the outer edge of the cell specified by the processing unit 11 based on the first image, and the circumference of the circle f2 indicated by the diagonal line inside the circle f1 is shown in the third image, and based on this, the outer edge of the nucleus identified by the processing unit 11 is shown. In the two-dimensional composite image, the X direction and the Y direction that are orthogonal to each other are set.

The processing unit 11 identifies the range x1 in the X direction and the range y1 in the Y direction in which the cells exist based on the synthetic image, and the intersection of the center line of the range x1 in the X direction and the center line of the range y1 in the Y direction is specified as the center position z1 of the cell, and the coordinates of the center position z1 of the cell are calculated. The processing unit 11 also specifies the range x2 in the X direction and the range y2 in the Y direction in which the nucleus exists based on the composite image, and the intersection of the center line of the range x2 in the X direction and the center line of the range y2 in the Y direction is specified as the center position z2 of the nucleus, and the coordinates of the center position z2 are calculated. The processing unit 11 calculates the nuclear uneven distribution value defined by the distance d between the two center positions z1 and z2 using the coordinates of the cell center position z1 and the coordinates of the nucleus center position z2.

Note that the X direction or the Y direction may be matched with the direction of the sample flow. Although, for convenience of explanation, the method of calculating the nuclear uneven distribution value in the processing unit 11 has been described using the composite image of the first image and the third image, it is sufficient if the distance between the center coordinates of the cell in the first image and the center coordinates of the nucleus in the third image can be obtained, and it is not essential to create a composite image.

FIG. 4 is a graph plotting the nuclear uneven distribution value and area of each cell contained in the sample 20a prepared from one sample (bone marrow fluid) in two-dimensional coordinates with the nuclear uneven distribution value as the parameter on the horizontal axis and the cell area as the parameter on the vertical axis. In FIG. 4, the vertical axis indicates the number of pixels determined to be a part of the cell, and one pixel matches 0.25 $\mu$m.$^2$. The horizontal axis indicates the distance d between the center position z1 of the cell and the center position z2 of the nucleus in units of $\mu$m. In the present embodiment, in FIG. 4, a plurality of cells included in a test region shown as a Plasma Cell Gate in which both the cell area and the nuclear uneven distribution value are large are set as test cells, and bright spot analysis (FISH images) is performed on the two second images only on the test cells. Next, the reasoning will be explained.

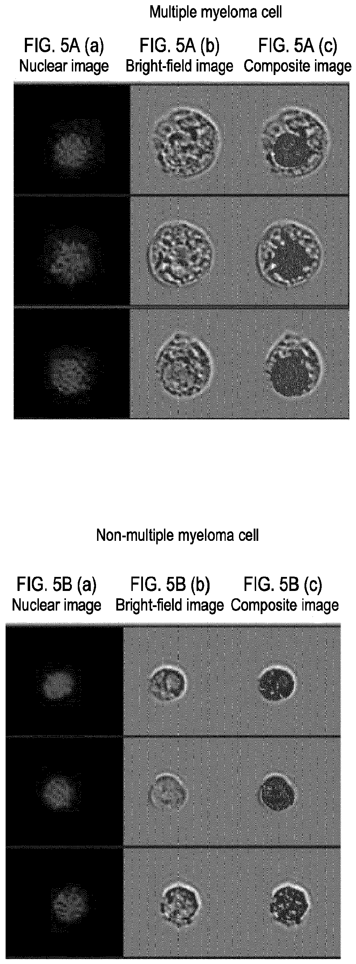

FIG. 5A is a diagram showing a nuclear image (a), a bright field image (b), and a composite image (c) thereof in multiple myeloma cells (cancerous plasma cells). FIG. 5B is a diagram showing a nuclear image (a), a bright field image (b), and a composite image (c) thereof in non-multiple myeloma cells. Note that the non-multiple myeloma cells are cells that are neither plasma cells nor cancerous myeloma cells. In the composite image of FIG. 5A (c) and the composite image of FIG. 5B (c), the nuclear region is shown in black. As shown in FIGS. 5A (b) and 5B (b), multiple myeloma cells are often larger than non-multiple myeloma cells.

As shown in FIGS. 5A (c) and 5B (c), since the nuclei of multiple myeloma cells are located off the center of the cells, whereas the nuclei of non-multiple myeloma cells are located in the center of the cells, the nuclear uneven distribution value of multiple myeloma cells is often greater than the nuclear uneven distribution value of non-multiple myeloma cells. As described above, multiple myeloma cells are often cells having a large cell area and a large uneven distribution value of nuclei as compared with non-multiple myeloma cells. Therefore, in the two-dimensional coordinate graph in FIG. 4, multiple myeloma cells are analyzed by bright spot analysis in the cells in the examination area, using the Plasma Cell Gate, which has a large cell area and nuclear uneven distribution value, as the examination area so that bright spot analysis can be performed efficiently.

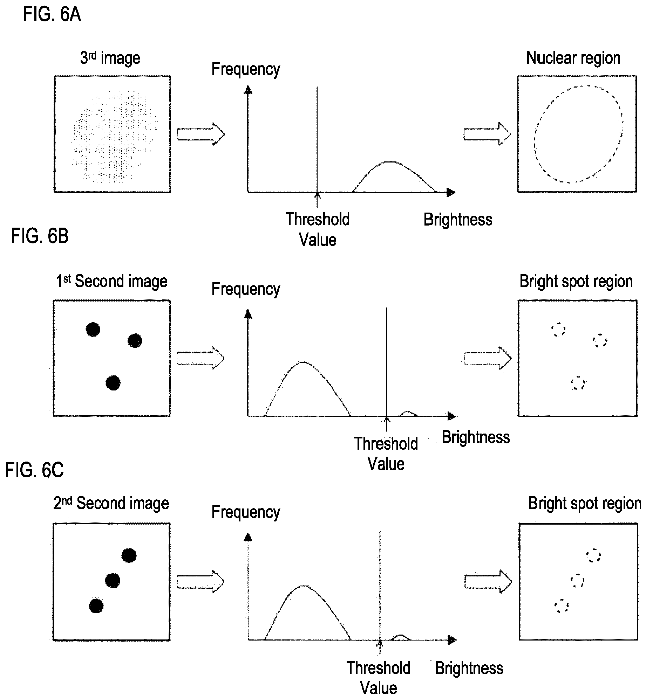

Next, in addition to FIG. 2, a method for extracting fluorescent bright spots and nuclear regions will be described with reference to FIG. 6. Note that the third image of FIG. 6A, the first second image of FIG. 6B, and the second second image of FIG. 6C are acquired from the same region of the sample 20a flowing through the flow cell 110. In the present embodiment, the fluorescent bright spot is a spot of fluorescence emitted by the fluorescent dye of the nucleic acid probe, and is a region where the brightness (pixel value) of each pixel configuring the image is higher than the brightness of the surrounding pixels. The fluorescent bright spot is extracted by a binarization treatment described later.

In the example shown in FIG. 2, the first second image has three bright spots of the first fluorescence having a wavelength $\lambda$21, and the second second image has three bright spots of the second fluorescence having a wavelength $\lambda$22. The third image is a fluorescence image corresponding to the third fluorescence of the wavelength $\lambda$23 indicating the region of the nucleus. The composite image is an image in which two second images are superimposed, and is an image for determining a fusion bright spot, which will be described later. In the flow path 111 of the flow cell 110, the cells of the sample 20a flow at intervals from each other. Therefore, when this is imaged by the image capture 154, as shown in FIG. 2, three fluorescence images and a bright field image are obtained for each cell.

When the third image shown in FIG. 6A is acquired, the processing unit 11 creates a graph of the brightness and frequency as shown in the center of FIG. 6A based on the brightness in each pixel on the third image. The frequency on the vertical axis is the number of pixels. The processing unit 11 sets a threshold value of brightness, which is a boundary between the nuclear region and the background, based on this graph. Then, the processing unit 11 performs a binarization process representing the third image by the pixels having a brightness below the threshold value and the pixels having a brightness higher than the threshold value, and extracts the range in which the pixels having the brightness higher than the threshold value are distributed as the nuclear region. The circumference of the circle drawn by the dotted line in the figure on the right side of FIG. 6A indicates the outer edge of the extracted nuclear region.

When the first second image shown in FIG. 6B is acquired, the processing unit 11 creates a graph of brightness and frequency as shown in the center of FIG. 6B based on the brightness in each pixel on the first image. Similar to the processing of the third image, the processing unit 11 sets a brightness threshold value in this graph, and extracts a range in which pixels having a brightness larger than the threshold value are extracted as a region of fluorescent bright spots. The circumference of the circle drawn by the dotted line in the figure on the right side of FIG. 6B indicates the outer edge of the region of the extracted fluorescent bright spot.

When the second second image shown in FIG. 6C is acquired, the processing unit 11 creates a graph of brightness and frequency as shown in the center of FIG. 6C based on the brightness in each pixel on the second image. Similar to the processing of the third image, the processing unit 11 sets a brightness threshold value in this graph, and extracts a range in which pixels having a brightness larger than the threshold value are extracted as a region of fluorescent bright spots. The circumference of the circle drawn by the dotted line in the figure on the right side of FIG. 6C indicates the outer edge of the region of the extracted fluorescent bright spot.

The processing unit 11 also may extract the nuclear region from the third image by calculation according to the above procedure without creating the graph as shown in FIGS. 6A to 6C, the region of the fluorescent bright spot may be extracted from the first image and the second image. Although the processing unit 11 extracts the nuclear region from the third image, the nuclear region also may be detected based on the bright field image. In that case, it is possible to omit the acquisition of the third image.

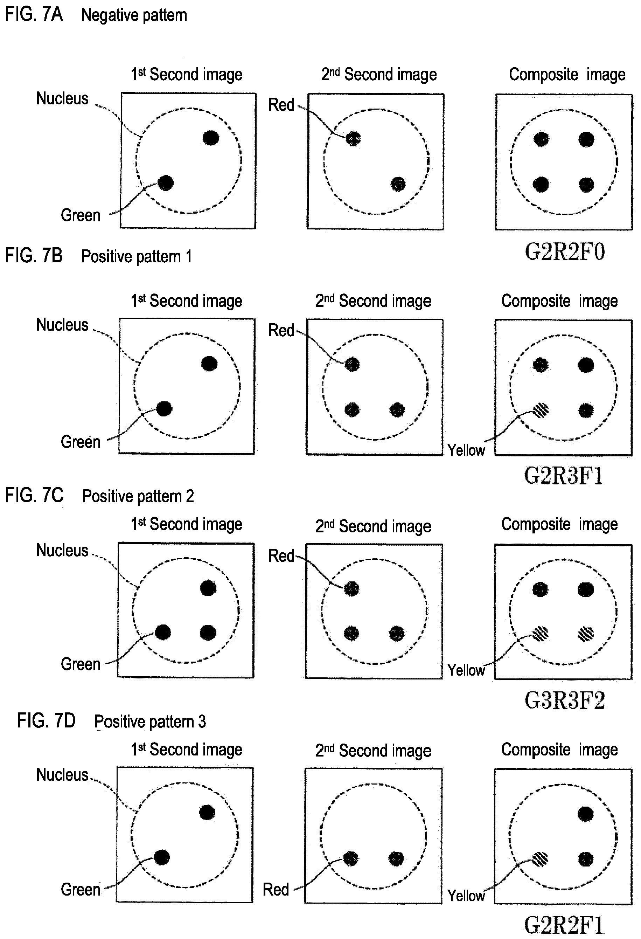

Next, the classification of cells based on the bright spot pattern by the processing unit 11 will be described with reference to FIG. 7. Note that FIG. 7 is a schematic diagram showing a simplified bright spot pattern; in this simplified bright spot pattern, when cells are negative, two bright spots corresponding to the first gene appear in the first second image, and two bright spots corresponding to the second gene appear in the second second image.

FIG. 7A shows an example of arrangement of fluorescent bright spots of negative cells without chromosomal abnormalities, that is, negative pattern, and FIGS. 7B to 7D show arrangement of fluorescent bright spots of positive cells having chromosomal abnormalities, that is, a positive pattern. The first second image and the second second image of FIG. 7 are superposed images of a third image showing a nuclear region, and the composite image is an image obtained by superimposing two second images and a third image. The fluorescence image includes a first second image, a second second image, a third image, and a composite image. Hereinafter, the bright spot of the first fluorescence configuring the first second image is referred to as a "first bright spot", and the bright spot of the second fluorescence configuring the second second image is referred to as a "second bright spot".

FIG. 7 illustrates the alphabet of "GRF" and the numbers of "0 to 3" as the pattern of fluorescence bright spots. The "G" of the fluorescence bright spot pattern indicates the green first bright spot in the composite image, and the "R" indicates the red second bright spot in the composite image. Further, "F" indicates a yellow fusion bright spot in the composite image. The numbers immediately following G, R, and F indicate the number of bright spots of G, R, and F included in the composite image, respectively.

For example, in the case of the negative pattern "G2R2F0" shown in FIG. 7A, there are two first bright spots in the first second image, two second bright spots in the second second image, and a composite image shows that the fusion bright spot is 0. Similarly, in the case of the positive pattern "G2R3F1" shown in FIG. 7B, there is one first bright spot in the first second image, three second bright spots in the second second image, and a composite image shows that the fusion bright spot is 1.

In the example shown in FIG. 7A, when no chromosomal abnormality such as fusion of the first locus and the second locus has occurred, each gene is paired in one nucleus, and each gene exists independently. Therefore, the first second image has two first bright spots in one nuclear region, and the second second image has two second bright spots in the nuclear region. Then, when the first second image and the second second image captured with the same size are superimposed and combined, the two first bright spots and the two second bright spots are generated in the composite image, and will exist in one nuclear region without overlapping. The pattern of fluorescence bright spots in which the first bright spot and the second bright spot do not overlap each other in the nuclear region as shown in FIG. 7A is a negative pattern in which no chromosomal abnormality is observed.

In the example shown in FIG. 7B, a part of the second gene is fused with the first gene by translocation, and two first bright spots are present in the nucleus in the first second image, and there are three second bright spots in the nucleus of the second second image. In this case, when the first second image and the second second image are combined, one first bright spot, two second bright spots, and one fusion bright spot in which the first bright spot and the second bright spot overlap each other exist in one nucleus in the composite image. The pattern of fluorescence bright spots as shown in FIG. 7B is a positive pattern in which a chromosomal abnormality is observed.

The fused bright spot generated by overlapping the first bright spot and the second bright spot appears yellow in the composite image. The presence or absence of fused bright spots, along with the number of fluorescence bright spots, is an important classification index in the FISH test.

In the example shown in FIG. 7C, a part of the first gene is fused with the second gene and a part of the second gene is also fused with the first gene due to the translocation, and there are three first bright spots in the nucleus of the first second image, and three second bright spots are present in the second second image. In this case, when the first second image and the second second image are combined, one first bright spot, two second bright spots, and one fusion bright spot in which the first bright spot and the second bright spot overlap each other exist in one nucleus in the composite image. The pattern of fluorescence bright spots as shown in FIG. 7C is a positive pattern in which a chromosomal abnormality is observed.

In the example shown in FIG. 7D, the translocation causes the entire second gene to fuse with the first gene, the first second image has two first bright spots in the nucleus, and the second second image has two second bright spots in the nucleus. In this case, when the first second image and the second second image are combined, one first bright spot, one second bright spot, and one fusion bright spot in which the first bright spot and the second bright spot are overlap each other exist in one nucleus occurs in the composite image. The pattern of fluorescence bright spots as shown in FIG. 7D is a positive pattern in which a chromosomal abnormality is observed.

According to the FISH method described above, it is possible to determine whether each cell is a positive cell having a chromosomal abnormality cell by cell, based on the number of red and green fluorescence bright spots and the number of fusion bright spots in the composite image of the first second image and the second second image. The processing unit 11 counts the number of first bright spots in the first second image of each cell, the number of second bright spots in the second second image, and the number of fusion bright spots where the first bright spot and the second bright spot overlap each other when the first second image and the second second image are combined. The processing unit 11 determines whether a cell is a positive cell or a negative cell by classifying the cells into any of FIGS. 7A to 7D based on, for example, the number of counted first bright spots (G), the number of second bright spots (R), and the number of fusion bright spots (F).

In the case of the present embodiment, a plurality of bright spot patterns wherein the first gene is the IGH gene and the second gene is the FGFR3 gene are stored in the storage unit 12. More specifically, when the first gene is the IGH gene and the second gene is the FGFR3 gene, a plurality of bright spot patterns corresponding to the negative pattern "G2R2F0" and the positive patterns "G2R3F1", "G3R3F2", and "G2R2F1" exemplified in FIG. 7 are stored in the storage unit 12. The processing unit 11 counts the number of first bright spots (G), number of second bright spots (R), and number of fusion bright spots (F) from the first second image, the second second image, and the composite image of the two second images of the cell to be analyzed, compares the the result with the negative patterns and the positive patterns stored in the storage unit 12, and when the result of the count corresponds to a positive pattern, determines that the cell is a positive cell, and when the count result corresponds to a negative pattern, determines that the cell is a negative cell.

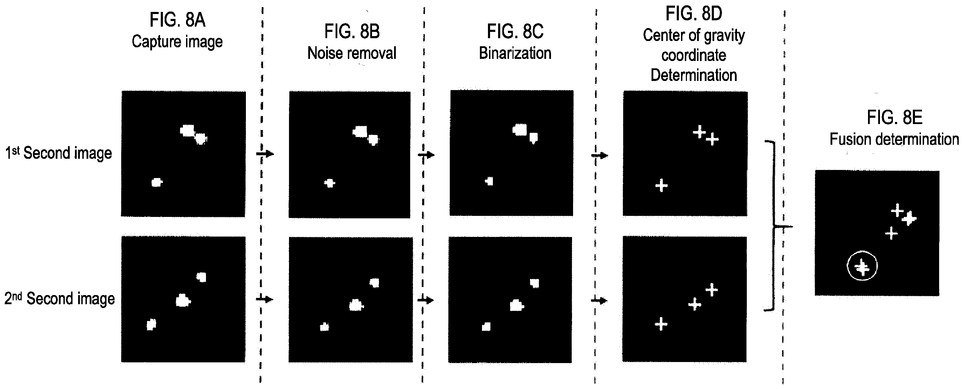

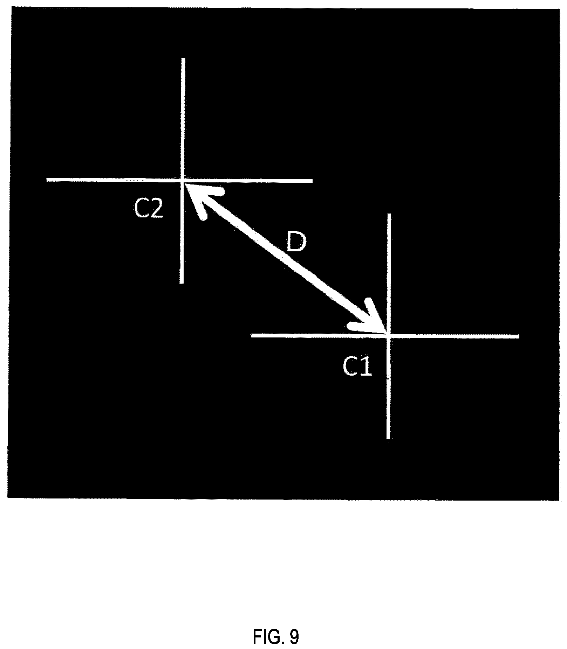

Next, a method of extracting bright spots and determining fusion bright spots by the processing unit 11 will be described with reference to FIGS. 8 and 9. FIG. 8A shows a first second image and a second second image obtained by the image capture unit 154. As shown in FIG. 8B, the processing unit 11 removes noise from the fluorescence image (two second images). Since noise is generally present in a fluorescence image, the processing unit 11 executes noise removal processing by using, for example, a noise removing means such as a top hat filter.

The processing unit 11 performs binarization processing on the noise-removed fluorescence image as shown in FIG. 8C. The setting of the threshold value in the binarization process is as described with reference to FIG. 6. As shown in FIG. 8D, the processing unit 11 calculates the coordinates of the center of gravity of the fluorescence bright spots extracted by the binarization process. The center of gravity coordinates mean the coordinates of the geometric center of gravity of the fluorescence bright spot, and can be calculated based on a predetermined equation. As shown in FIG. 8E, the processing unit 11 determines whether the first and second bright spots that are close to each other are fused bright spots from the distance D between the centers of gravity of the first bright spot and the second bright spot. The first and second bright spots existing in the circle drawn in the composite image of FIG. 8E are close to each other, and determined as the fusion bright spot generated due to the translocation of the IGH gene or the FGFR3 gene.

FIG. 9 is a schematic diagram for explaining in detail the fusion determination of FIG. 8E. As shown in FIG. 9, the processing unit 11 calculates the distance D between the centers of gravity C1 of the first bright spot and the center of gravity coordinates C2 of the second bright spot, and compares the distance D between the centers of gravity and the threshold value. As the threshold value, a distance corresponding to the diameter of one standard fluorescence bright spot is used. A fixed value is used for the threshold value regardless of the sample. Note that the threshold value may not be a fixed value, and the threshold value may be set variably depending on the sample. For example, a representative value thereof may be calculated from the diameters of the plurality of fluorescence bright spots extracted from the plurality of cells contained in the sample 20a, and the representative value may be applied to the threshold value. The representative value may be an average value, a median value, or a mode value.

When the distance D between the centers of gravity is equal to or less than the threshold value, the processing unit 11 determines that the first bright spot and the second bright spot are fused. When the distance D between the centers of gravity is larger than the threshold value, the processing unit 11 determines that the first bright spot and the second bright are not fused. The processing unit 11 calculates the distance D between the centers of gravity of all the first bright spot existing in the nuclear region from each of the second bright spots, and makes a fusion determination by comparing with the threshold value. The processing unit 11 counts the number of fusion bright spots for each cell. Note that although FIGS. 8E and 9 show that the fusion determination is performed on the composite image of the first second image and the second second image for convenience of explanation, the creation of a composite image is unnecessary insofar as it is sufficient if the distance between the center of gravity coordinates of each bright spot in the first second image and the center of gravity coordinates of each bright spot in the second second image are obtained for the fusion determination by the processing unit 11.

The fusion determination of whether the first bright point and the second bright point overlap may be performed by using the distance between the center point of the first bright point and the center point of the second bright point instead of the distance D between the centers of gravity. In the present specification, the center point means the point having the highest brightness among the fluorescence bright spots and the pixel having the highest brightness. When the distance between the center points of the first bright spot and the second bright spot is equal to or less than a predetermined threshold value, the processing unit 11 may determine that the first bright spot and the second bright spot are fused, and when the distance between the center points is larger than a predetermined threshold value, it may be determined that fusion is not present.

Further, it is also possible to make a fusion determination by comparing the ratio of the regions where the first bright spot and the second bright spot overlap each other, that is, the ratio of the pixels configuring the first bright spot at the same position (same coordinates) as the pixels configuring the second bright spot, with the threshold value.

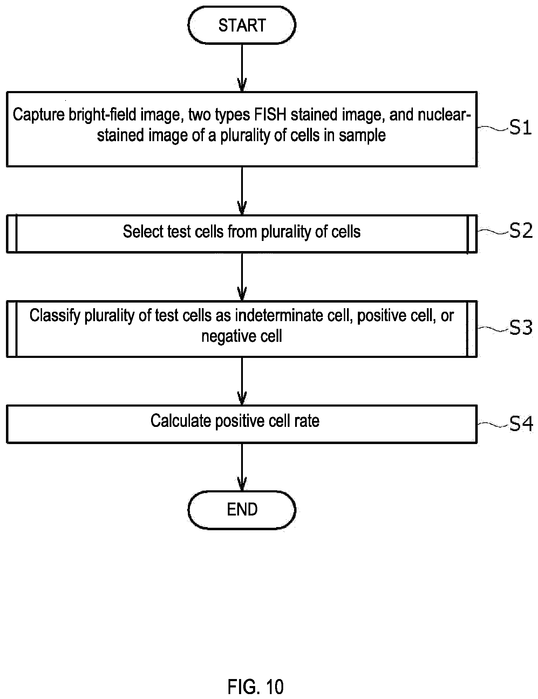

Next, the fluorescence image analysis process executed by the processing unit 11 in the present embodiment will be described with reference to FIGS. 10 to 12. FIG. 10 is a flowchart showing an example of a procedure of fluorescence image analysis processing executed by the processing unit 11. The process shown in FIG. 10 is executed by the processor of the processing unit 11 executing the program stored in the storage unit 12. When the sample 20a obtained by the operator performing pretreatment using the pretreatment unit 20 is set in the fluorescence image analyzer 10 and an instruction to start measurement is given via the input unit 14, the processing unit 11 starts the series of the analysis process.

In step S1, the processing unit 11 controls the fluid circuit unit 15 of the fluorescence image analyzer 10 to flow the sample 20a into the flow cell 110. The processing unit 11 causes the light sources 121 to 124 to emit light. In this way the cells in the sample 20a flowing through the flow cell 110 are irradiated with light. The processing unit 11 causes the image capture unit 154 to capture a fluorescence image and a bright-field image of cells. In this way a fluorescence image and a bright-field image (first image) are acquired for each cell. As the fluorescence image, a first second image corresponding to a first fluorescence, a second second image corresponding to a second fluorescence, and a third image (nuclear staining image) corresponding to a third fluorescence are imaged. The processing unit 11 stores the three fluorescence images and bright-field image for each cell in the storage unit 12 in association with the cell IDs assigned in the order of imaging to identify each cell.

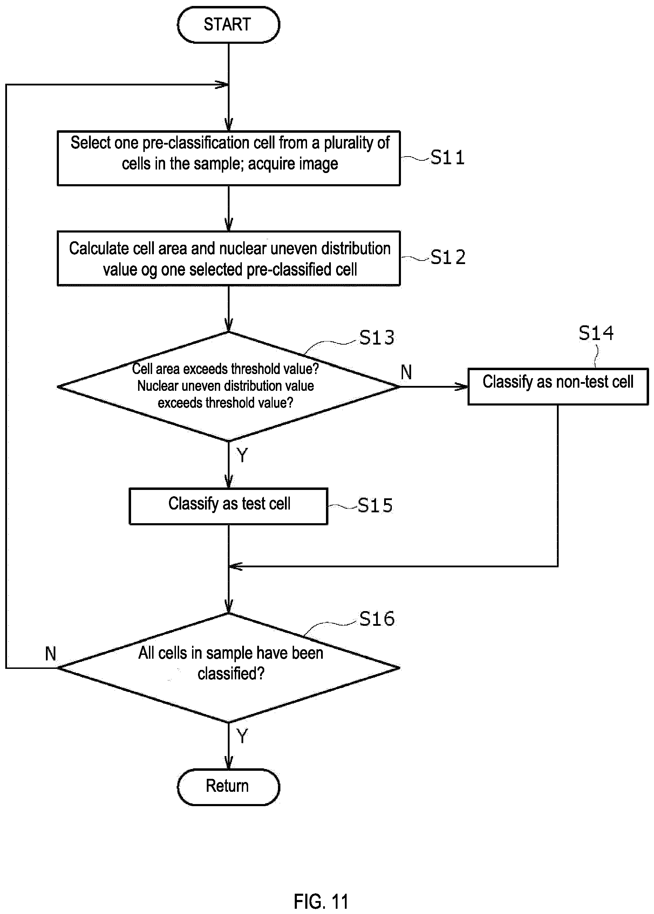

In step S2, the processing unit 11 analyzes the first image and the third image, and the plurality of cells in the sample 20a (in this embodiment, all the cells in the sample 20a) having a large proportion of plasma cells and myeloma cells are selected as a plurality of test cells. More specific processing performed by the processing unit 11 in step S2 will be described in detail later with reference to FIG. 11.

When step S2 is completed, the process proceeds to step S3. In step S3, the processing unit 11 analyzes the fluorescence images (first second image, second second image) of the plurality of cells classified into the test cells in step S2, and classifies the cells in the fluorescence image. as positive cells, negative cells, or indeterminate cells.

When step S3 is completed, the process proceeds to step S4. In step S4, the processing unit 11 calculates the positive cell rate for the gene fusion of target object based on the number of positive cells and the number of negative cells acquired in step S3 using the following equation (1). The positive cell rate is an example of information related to the rate of cells undergoing gene fusion.

$$\text{Number of positive cells} / (\text{number of negative cells} + \text{number of positive cells}) \ldots (1)$$

In equation (1), the indeterminate cells corresponding to the unclear fluorescence image are excluded, and the positive cell rate is calculated only by the number of negative cells and the number of positive cells. By doing so, it is possible to output a more accurate positive cell rate.

Next, the processes of steps S2 and S3 in FIG. 10 will be described in detail. FIG. 11 is a flowchart showing an example of the detailed procedure of the classification process of step S2 of FIG. 10. With reference to FIG. 11, in the classification process of the test cells and the non-test cells, first, in step S11, the processing unit 11 uses the cell ID as a key to select one cell before classification from a plurality of cells in the sample, and the first image and the third image stored in association with the selected cells are acquired from the storage unit 12 (read from the storage unit 12). Here, the cell selected first is the cell corresponding to the image captured earliest, but the cell is not limited to this.

In the next step S12, the processing unit 11 calculates the cell area and the nuclear uneven distribution value for the selected unclassified 1 cell. Specifically, the processing unit 11 calculates the cell area by counting the number of pixels in which a part of the cell is shown in the bright-field image (first image). The processing unit 11 also calculates the nuclear uneven distribution value by the method described with reference to FIG. 3 based on the bright-field image and the nuclear stained image (third image).

In the next step S13, the processing unit 11 determines whether the calculated cell area is larger than the predetermined value (threshold area), and the calculated nuclear uneven distribution value is larger than the predetermined value (threshold nuclear uneven distribution value). Although the threshold area is set to an area of 90 pixels and the threshold nuclear uneven distribution value is set to 1.2 $\mu$m in the example shown in FIG. 4, the threshold area and the threshold nuclear uneven distribution value are not limited to these values. Although the upper limit value of the cell area and the upper limit value of the nuclear uneven distribution value are set as the conditions for affirmative determination in step S13 in the example shown in FIG. 4, these upper limit values may not be set.

If an affirmative determination is made in step S13, the processing unit 11 classifies the selected cell 1 as a test cell in step S15. Specifically, in step S15, the processing unit 11 associates the cell ID of the selected cell with a label indicating that it is a test cell and stores it in the storage unit 12. When step S15 is completed, the process proceeds to step S16, and the processing unit 11 determines whether all the cells in the sample have been classified. If a negative determination is made in step S16, steps S11 and subsequent steps are repeated, and if an affirmative determination is made in step S16, the process of step S2 ends, and the process returns to the process of step S3 of FIG. 10.

On the other hand, if a negative determination is made in step S13, the processing unit 11 classifies the selected cell 1 as a non-test cell in step S14. Specifically, in step S14, the processing unit 11 associates the cell ID of the selected cell with a label indicating that it is a non-test cell and stores it in the storage unit 12. When step S14 is completed, the process proceeds to step S16.

Regarding the selection of cells in step S11, the order of cell selection may be the order stored in the storage unit 12 instead of the imaging order. In steps S11 to S16 in the flow of FIG. 11, it is not necessary to select the next cell after the processing of step S16 is completed for the cell selected in step S11, and a plurality of cells may be processed in parallel. For example, the next cell may be selected at with the timing of starting the process of step S12 for the cell selected in step S11. In step S2, the processing unit 11 also may perform any treatment that can classify all the cells in the sample 20a into test cells and non-test cells. As described with reference to FIGS. 5A and 5B, step S2 is performed to efficiently extract plasma cells and myeloma cells.

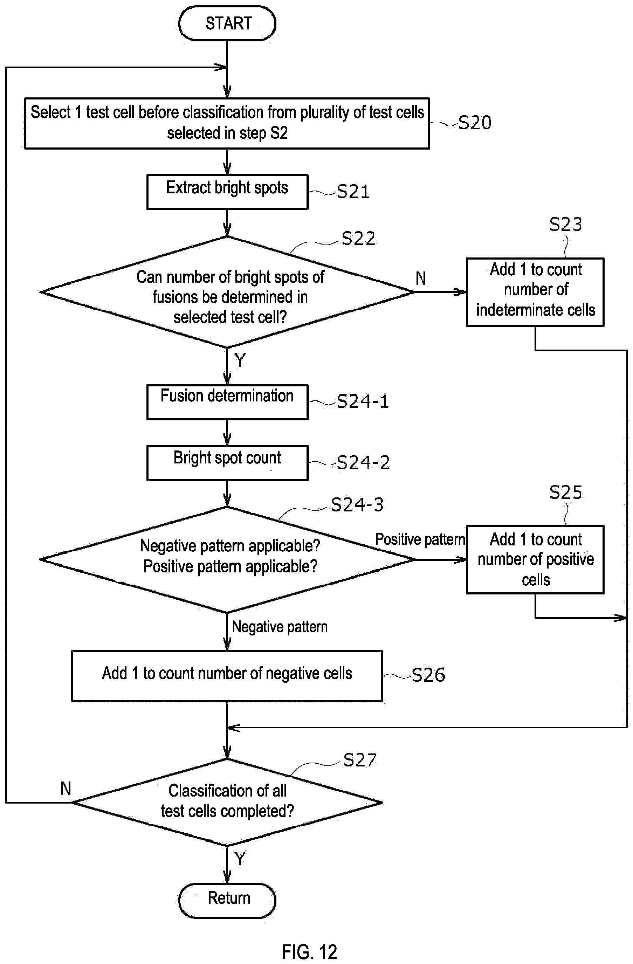

FIG. 12 is a flowchart showing an example of the detailed procedure of the classification process of step S3 of FIG. 10. With reference to FIG. 12, in the classification process of positive cells, negative cells, and indeterminate cells, first, in step S20, the processing unit 11 selects one test cell before classification from the plurality of test cells selected in step S2 of FIG. 10, and acquires (reads from the storage unit 12) a second image (both the first second image and the second second image) stored in association with the selected cell from the storage unit 12. Note that the processing unit 11 can identify a cell whose label indicates that it is a test cell associated with a cell ID as a test cell.

In step S21, the processing unit 11 removes noise from the captured image and performs binarization processing to extract a fluorescence bright spot, as described with reference to FIGS. 6 and 8. The first bright spot is extracted from the first second image that has been binarized, and the second bright spot is extracted from the second second image as the fluorescence bright spots. Then, in the next step S22, it is determined whether the processing unit 11 can determine the number of bright spots in the second image (both the first second image and the second second image) with respect to the selected 1 test cell. When the processing unit 11 determines negative in step S22, the process proceeds to step S23, and the processing unit 11 increases the count number of the indeterminate cells stored in the storage unit 12 by 1, and updates the indeterminate cells to the storage unit 12. Note that before the start of step S3 in FIG. 10, the storage unit 12 stores 0 as the number of in-determinate cells.

The processing unit 11 determines whether the number of bright spots can be determined as follows. Specifically, when the shape of the fluorescence bright spot diverges from a perfect circle such as an elliptical shape, the processing unit 11 determines that the fluorescence image is not subject to classification by software, and classifies the fluorescence image as non-target. The first bright spot and the second bright spot usually have a shape approximating a perfect circle, but may have a shape divergent from a perfect circle such as an elliptical shape. One reason this is so is that the moving speed of cells flowing through the flow path 111 of the flow cell 110 and the charge transfer speed of the line sensor of the TDI camera do not match, causing the fluorescence bright spot to elongate or contract in the Y direction, which is the direction of the sample flow. In this case, it is difficult to obtain accurate center of gravity coordinates of the fluorescence bright spot, and the software may make an erroneous fusion determination; therefore, the cell corresponding to such a fluorescence image is determined to be an indeterminate cell.

In this determination, for example, when the ratio (Y / X) of the length of the fluorescence bright spot in the Y direction to the length in the X direction orthogonal to the Y direction exceeds a predetermined threshold value, the processing unit 11 classifies the cell as a non-target cell. Note that instead of the length ratio (Y / X), another index indicating the shape of the fluorescence bright spot, such as the circularity of the fluorescence bright spot, also may be used.

When the size of the fluorescence bright spot exceeds a predetermined threshold value, the processing unit 11 excludes the fluorescence image from the classification by software, and classifies the fluorescence image as a non-target. Although the first bright spot and the second bright spot are usually of the same size, there may be cases of only a part of the fluorescence bright spots may be imaged in a large size. The reason for this is that the fluorescent labeling site of the cell may be present at a distance in the depth direction along the optical axis of the TDI camera. In this case, it is difficult to obtain the accurate center of gravity coordinates of the fluorescence bright spot, and software may make an erroneous fusion determination, so such a fluorescence image is excluded from cell classification. For example, when the area of the fluorescence bright spot exceeds a predetermined threshold value, the processing unit 11 classifies the fluorescence image as a non-target.

The processing unit 11 excludes the fluorescence image from the classification by software when the brightness of the fluorescence bright spot is lower than a predetermined threshold value, and classifies the fluorescence image as a non-target. Although the maximum value of the luminance value in the two second images is usually about the same, it is conceivable that the luminance may be greatly reduced due to the influence of preprocessing and the like. The sample 20a is prepared by a pretreatment for fluorescently labeling the target site of the cells, but it is generally difficult to perform a uniform pretreatment for all cells, and so-called poorly stained cells that are not fluorescently labeled to the desired level can occur. For example, when at least one of the maximum value of the luminance value of the first second image and the maximum value of the bright spot of the second image is less than a predetermined threshold value, the processing unit 11 classifies the fluorescence image as non-target.

When step 23 is completed, the process proceeds to step S27, and the processing unit 11 determines whether all the classifications of the plurality of test cells have been completed. If the processing unit 11 determines affirmatively in step S27, the processing unit 11 ends the processing of step S3 in FIG. 10 and returns the processing to step S4. On the other hand, if a negative determination is made in step S27, the processing unit 11 proceeds to step S20, newly selects a test cell before classification of the 1, and performs the processing of step S21 and thereafter on the selected test cell 1.

On the other hand, if an affirmative determination is made in step S22, the process proceeds to step S24-1, and the processing unit 11 selects from the composite image of the second image (both the first second image and the second second image), and determines whether gene fusion has occurred in the test cell of 1. This fusion determination is performed based on the method described with reference to FIGS. 8 and 9. The processing unit 11 counts the first bright spot, the second bright spot, and the fusion bright spot for the selected 1 test cell in step S24-2. In step S24-3, the processing unit 11 determines whether the count value in step S24-2 corresponds to either a positive pattern or a negative pattern stored in the storage unit 12. This determination is made based on the method described with reference to FIG. 7. If it is determined in step S24-3 that the positive pattern is applicable, the process proceeds to step S25, the cell ID of the cell is associated with a label indicating that the cell is a positive cell and stored in the storage unit 12. The processing unit 11 also increases the count number of positive cells stored in the storage unit 12 by 1, and stores the updated number of positive cells in the storage unit 12. Note that before the start of step S3 in FIG. 10, the number of positive cells is stored as 0 in the storage unit 12. When step S25 is completed, the process proceeds to step S27.

On the other hand, if it is determined in step S24-3 that the pattern corresponds to a positive pattern, the process proceeds to step S26, and the cell ID of the cell is associated with a label indicating that it is a negative cell and stored in the storage unit 12. The processing unit 11 also increases the count number of negative cells stored in the storage unit 12 by 1, and stores the updated number of negative cells in the storage unit 12. Note that before the start of step S3 in FIG. 10, the number of negative cells is stored as 0 in the storage unit 12. When step S26 is completed, the process proceeds to step S27. Note that the determination in step S24-3 is not limited to the above method of comparing the count values of the first bright spot, the second bright spot, and the fusion bright spot with the positive pattern and the negative pattern; for example, the processing unit 11 may count the fusion bright spots, determine positive if there are one or more fusion bright spots, and determine negative if there are no fusion bright spots.

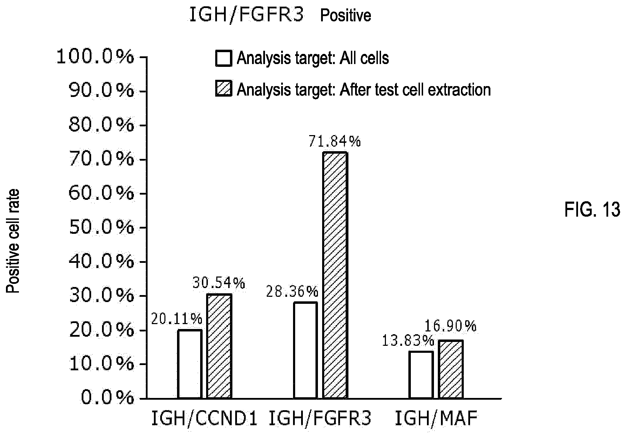

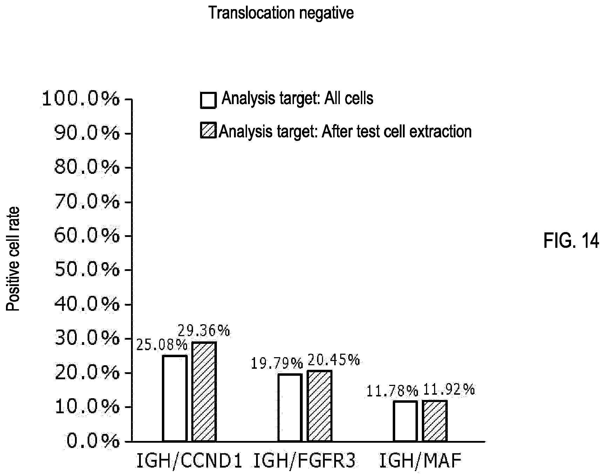

FIG. 13 is a graph showing the calculation results when rates were calculated for positive cell rates of the IGH / CCND1 fusion gene, the IGH / FGFR3 fusion gene, and the IGH / MAF fusion gene in the bone marrow sample (sample 1) of a multiple myeloma patient having an IGH translocation using the method of the present embodiment described above. Plasma cells were not isolated from Sample 1. Sample 1 is a positive sample in which the IGH / FGFR3 fusion gene is generated. In sample 1, IGH / CCND1 fusion gene and IGH / MAF fusion gene did not occur. FIG. 14 is a graph showing the calculation results when rates were calculated for positive cell rates of the IGH / CCND1 fusion gene, the IGH / FGFR3 fusion gene, and the IGH / MAF fusion gene in the bone marrow sample (sample 2) of a multiple myeloma patient having no IGH translocation using the method of the present embodiment described above. Plasma cells were not isolated from sample 2. Sample 2 is a negative sample in which the IGH / CCND1 fusion gene, the IGH / FGFR3 fusion gene, and the IGH / MAF fusion gene did not occur. Note that, in FIGS. 13 and 14, as a comparative example, step S2 in FIG. 10 is omitted, all the cells in the sample 20a are treated with steps S3 and S4 in FIG. 10 without selecting test cells, and the calculation result of calculating the positive cell rate is also shown. In FIGS. 13 and 14, the positive cell rate according to the method of this embodiment is represented by a bar graph with hatching, and the positive cell rate of the comparative example is represented by a bar graph without hatching. Note that in the following description the IGH / CCND1 fusion gene and the IGH / MAF fusion gene may be referred to as t(11; 14) and t(14; 16), respectively.

Regarding the positive cell rate calculated by the method of this embodiment, when the sample 1 which is a sample having an IGH translocation and the sample 2 which is a sample without an IGH translocation are compared, as shown in FIGS. 13 and 14 above, regarding the IGH / FGFR3 fusion gene, the positive cell rate of sample 1 is 71.84%, whereas the positive cell rate of sample 2 is 20.45%, and the difference in positive cell rate exceeds 50%. Therefore, it is possible to set a threshold value between 20.45% and 71.84%, and facilitate the diagnosis by generating and outputting the positive cell rate as an index for the diagnosis of multiple myeloma.

On the other hand, when the positive cell rate calculated by the method of the comparative example is compared between the sample 1 and the sample 2, as shown in FIGS. 13 and 14, the positive cell rate of the sample 1 is 28.36% with respect to the IGH / FGFR3 fusion gene, and the positive cell rate of sample 2 is 19.79%, and the difference in positive cell rate is less than 9%. In this way when the difference in positive cell rate between a sample having an IGH translocation and a sample not having an IGH translocation is small, the positive cell rate often cannot be used as an index for diagnosing multiple myeloma.

Regarding the IGH / FGFR3 fusion gene of sample 2, as shown in FIG. 14, the positive cell rate calculated by the method of this embodiment is 20.45%, and the positive cell rate calculated by the method of the comparative example is 19.79%. Since the sample 2 is a negative sample that does not generate the IGH / FGFR3 fusion gene, it is considered that about 20% of the negative cells were determined to be positive cells (hereinafter referred to as false positive determination). From here it can be seen, regarding the IGH / FGFR3 fusion gene of Sample 1, the positive cell rate of 71.84% calculated by the method of the present embodiment and 28.36% of the positive cell rate calculated by the method of the comparative example each include about 20% of false positive determinations. Here, regarding the IGH / FGFR3 fusion gene of sample 1, even when 20% is subtracted from the positive cell rate calculated by the method of this embodiment, the positive cell rate exceeds 50%, whereas when 20% is subtracted from the positive cell rate calculated by the method of the comparative example, the positive cell rate is below 9%. This is because, in the method of the present embodiment, the false positive determination does not affect the diagnosis of multiple myeloma and the diagnosis can be facilitated, whereas, in the method of the comparative example, the false positive determination has an effect and the diagnosis of multiple myeloma becomes difficult.

Regarding the IGH / FGFR3 fusion gene of sample 2, as shown in FIG. 14, the positive cell rate calculated by the method of this embodiment is 71.84%, whereas the positive cell rate calculated by the method of the comparative example is 28.36%. The numerator in the equation for calculating the positive cell rate (71.84%) calculated by the method of this embodiment is 574, and the denominator is 799. On the other hand, the numerator in the equation for calculating the positive cell rate (28.36%) calculated by the method of the comparative example is 3674, and the denominator is 12954. By setting the Plasma Cell Gate shown in FIG. 4, regarding the numerator in the calculation equation of the positive cell rate, it was shown that 574 cells were selected from 3674 cells, that is, the selection rate was 15.6%; as for the denominator, it is shown that 799 cells were selected from 12954 cells, that is, the selection rate was 6.2%. As described above, in the method of the present embodiment, by setting the Plasma Cell Gate, the cell selection rate in the numerator of the positive cell rate calculation equation becomes larger than the cell selection rate in the denominator, and the positive cells can be efficiently extracted.

For the IGH / CCND1 fusion gene of sample 1, as shown in FIG. 13, the positive cell rate calculated by the method of this embodiment is 30.54%, whereas the positive cell rate calculated by the method of the comparative example is 20.11%, and the difference in positive cell rate is less than 11%. As shown in FIG. 13, for the IGH / MAF fusion gene of sample 1, the positive cell rate calculated by the method of this embodiment is 16.90%, whereas the positive cell rate calculated by the method of the comparative example is 13.83%, and the difference in positive cell rate is less than 4%. As described above, in the method of the present embodiment, even if the Plasma Cell Gate shown in FIG. 4 is set, the output positive cell rate is as accurate as the case in which the Plasma Cell Gate is not set for the sample having no IGH translocation.

As described above, in the method of the present embodiment, the difference between the positive cell rate in the sample having the IGH translocation and the positive cell rate in the sample without the IGH translocation becomes large, and the positive cell rate can be output as an index for the diagnosis of multiple myeloma. Therefore, the diagnosis of the subject can be facilitated.

That is, according to the present embodiment, the fluorescence image analyzer 10, automatically a process of selecting a plurality of test cells belonging to a region in which cells to be tested (plasma cells and myeloma cells in this embodiment) are frequently contained from a plurality of cells in a sample based on the software. Therefore, even if the ratio of plasma cells and myeloma cells to be tested is small relative to all cells contained in sample 20a, the positive cell rate can be output without performing a treatment for isolating those cells from sample 20a.

Modification Example Related to Selection of Test Cells

In the above embodiment, the cell area and nuclear uneven distribution value as selection information were calculated using the bright-field first image and the nuclear stained third image, and plasma cells and myeloma cells, which are test cells, were efficiently extracted from these two images of selection information. However, the selection information regarding the selection of the test cells is not limited to the cell area and the uneven distribution value of the nucleus, and may be any other information regarding the characteristics of the cells. Alternatively, a plurality of test cells may be selected from a plurality of cells in the sample without using selection information. Next, a modified example relating to the selection of test cells will be described.

First Modification

As an image for extracting selection information, a first image which is a bright-field image and a third image which is a fluorescence image of nuclear staining may be used. Then, as the selection information, the cell area extracted from the first image and the nucleus area extracted from the third image may be adopted.

Next, a modified example 1 in which the positive cell rate was calculated by adopting the cell area and the nuclear area as selection information in one sample of follicular lymphoma (pancreatic) in which the IGH / BCL2 fusion gene is generated will be described.

In this modification 1, one sample 20a was produced using pancreatic fluid as a sample and included hybridizing a nucleic acid probe labeled with the first fluorescent dye and the target site in the nucleic acid of the sample 20a, hybridizing a nucleic acid probe labeled with a second fluorescent dye and a target site in the nucleic acid, and labeling the nucleus of each cell with a dye for nuclear staining as a third fluorescent dye.

The sample 20a is subjected to an imaging process in the same manner as in the above embodiment, and the first image (bright-field image), the two second images, and the third image (nuclear stained image) was obtained for each cell in the sample 20a.. Then, the cell area was calculated for each cell from the first image, and the nuclear area was calculated from the third image for each cell. The cell area can be calculated by the processing unit 11 specifying the number of pixels in which a part of the cell is shown in the bright-field image. The nuclear area can be calculated by the processing unit 11 specifying the number of pixels in which a part of the nucleus is shown in the nuclear-stained image.

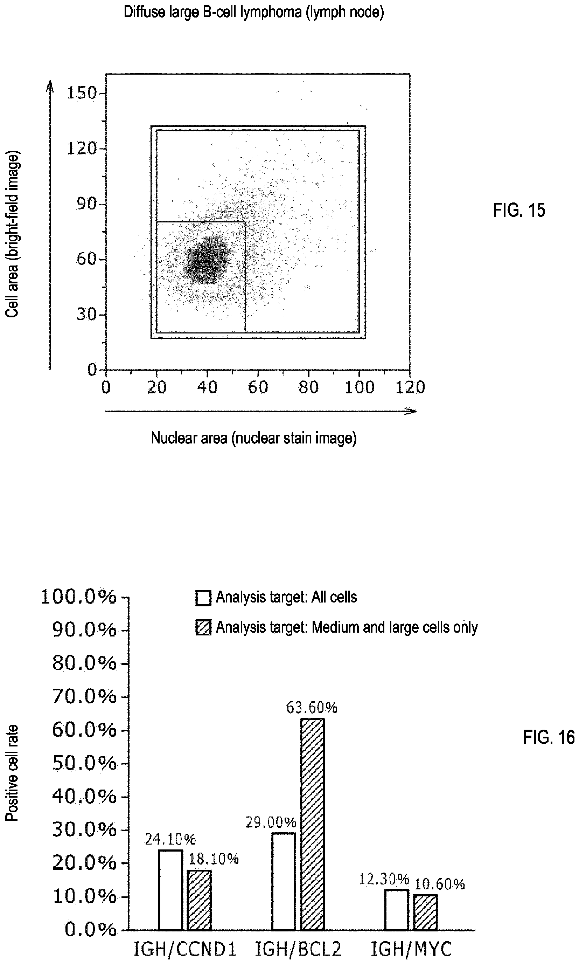

FIG. 15 is a graph in which the nuclear area and the cell area of each cell contained in the sample 20a are plotted on two-dimensional coordinates in which the nuclear area is a parameter on the horizontal axis and the cell area is a parameter on the vertical axis. In FIG. 15, the vertical axis shows the number of pixels of the portion determined to be a cell, and one pixel corresponds to 0.25 $\mu$m$^2$. The horizontal axis indicates the number of pixels in the portion determined to be the nucleus, and one pixel corresponds to 0.25 $\mu$m$^2$.

In the graph of FIG. 15 of modification 1, a cell existing in a region, excluding a region having a nuclear area of less than 55 pixels and a cell area of less than 80 pixels, was designated as a test cell, bright spot analysis was performed only on multiple test cells existing in the region, and cells in which one or more gene fusions of interest were present were designated as positive cells.

FIG. 16 is a graph which shows the calculation result at the time of calculation using the method of modification 1, describes above the positive cell rates of the IGH / CCND1 fusion gene, the IGH / BCL2 fusion gene, and the IGH / MYC fusion gene in spleen tissue specimens of patients with follicular lymphoma having an IGH translocation. This sample is a positive sample in which the IGH / BCL2 fusion gene is generated. In this sample, the IGH / CCND1 fusion gene and the IGH / MYC fusion gene did not occur. Note that, as a comparative example shown in FIGS. 13 and 14, step S2 in FIG. 10 is omitted, step S3 in FIG. 10 is omitted for all cells in sample 20a without selecting test cells, S4 treatment was performed to calculate the positive cell rate, and the calculation result are also shown also in this graph. In FIG. 16,The positive cell rate by the method of the present modification 1 is represented by a bar graph with hatching, and the positive cell rate of the comparative example is represented by a bar graph without hatching. Note that in the following description, the IGH / CCND1 fusion gene, the IGH / BCL2 fusion gene, and the IGH / MYC fusion gene are also referred to as t(11; 14), t(14; 18), and t(8; 14), respectively.

Regarding the IGH / BCL2 fusion gene of the sample, as shown in FIG. 16, the positive cell rate calculated by the method of this modification 1 is 79.6%, whereas the positive cell rate calculated by the method of the comparative example is 53.3%. The number of numerator in the equation for calculating the positive cell rate (79.6%) calculated by the method of this modification 1 is 9944, and the denominator is 12492. On the other hand, the number of the numerator in the equation for calculating the positive cell rate (53.3%) calculated by the method of the comparative example is 11238, and the denominator is 21079. By setting the region in the manner described in FIG. 15, the numerator for the equation for calculating the positive cell rate used 9944 cells selected from 11238 cells, that is, the selection rate was 88.4%, and the denominator used 12492 cells selected from 21079 cells, that is, the selection rate was 59.3%. As described above, in the method of the present modification 1, the cell selection rate in the numerator of the equation for calculating the positive cell rate becomes larger than the cell selection rate in the denominator due to the setting of the region described above shown in FIG. 15, and positive cells can be efficiently extracted.

Regarding the IGH / CCND1 fusion gene of this sample, as shown in FIG. 16, the positive cell rate calculated by the method of this modification 1 is 28.4%, whereas the positive cell rate calculated by the method of the comparative example is 26.0%, and the difference in positive cell rate is less than 3%. Regarding the IGH / MYC fusion gene of this sample, as shown in FIG. 16, the positive cell rate calculated by the method of this modification 1 is 19.4%, whereas the positive cell rate calculated by the method of the comparative example is 16.3%, and the difference in positive cell rate is less than 4%. In this way, in the method of the present modification 1, even if the above-described region shown in FIG. 15 is set, the above-described region shown in FIG. 15 is not set for the sample having no IGH translocation, and the positive cell rate can be output with the same accuracy.

As described above, in the method of the modification 1, the difference between the positive cell rate in the sample having the IGH translocation and the positive cell rate in the sample without the IGH translocation becomes large, and the positive cell rate can be output as an index for the diagnosis of follicular lymphoma. Therefore, the diagnosis of the subject can be facilitated.

Next, in another example using the method of modification 1, specifically, in the lymph node sample of a patient with diffuse large B-cell lymphoma having an IGH translocation, the cell area and the nuclear area are used as selection information; an example of adopting and calculating the positive cell rate will be described.

In this example, one sample 20a was produced using lymph node as a sample and included hybridizing a nucleic acid probe labeled with the first fluorescent dye and the target site in the nucleic acid of the sample 20a, hybridizing a nucleic acid probe labeled with a second fluorescent dye and a target site in the nucleic acid, and labeling the nucleus of each cell with a dye for nuclear staining as a third fluorescent dye.

The sample 20a was subjected to an imaging process, and the first image (bright-field image), the two second images, and the third image (nuclear stained image) was obtained for each cell in the sample 20a. Then, the cell area was calculated for each cell from the first image, and the nuclear area was calculated from the third image for each cell.

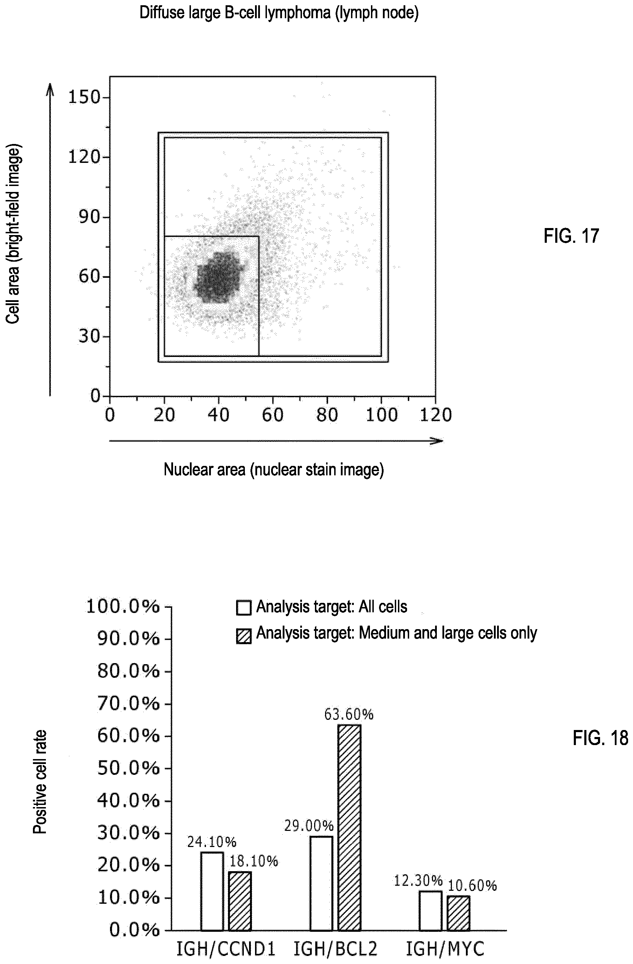

FIG. 17 is a graph in which the nuclear area and the cell area of each cell contained in the sample 20a are plotted on two-dimensional coordinates in which the nuclear area is a parameter on the horizontal axis and the cell area is a parameter on the vertical axis. In FIG. 17, the vertical axis shows the number of pixels of the portion determined to be a cell, and one pixel corresponds to 0.25 $\mu m^2$. The horizontal axis indicates the number of pixels in the portion determined to be the nucleus, and one pixel corresponds to 0.25 $\mu m^2$.

In the graph of FIG. 17 of this example, a cell existing in a region, excluding a region having a nuclear area of less than 55 pixels and a cell area of less than 80 pixels, was designated as a test cell, bright spot analysis was performed only on multiple test cells existing in the region, and cells in which one or more gene fusions of interest were present were designated as positive cells.

FIG. 18 is a graph showing the calculation result when the positive cell rates of the IGH / CCND1 fusion gene, the IGH / BCL2 fusion gene, and the IGH / MYC fusion gene in the lymph node specimens of patients with diffuse large B-cell lymphoma with IGH translocation were calculated using the method of another example of the above-mentioned modification 1. This sample is a positive sample in which the IGH / BCL2 fusion gene is generated. In this sample, the IGH / CCND1 fusion gene and the IGH / MYC fusion gene did not occur. Note that, as a comparative example shown in FIGS. 13 and 14, step S2 in FIG. 10 is omitted, step S3 in FIG. 10 is omitted for all cells in sample 20a without selecting test cells, S4 treatment was performed to calculate the positive cell rate, and the calculation result are also shown also in this graph. In FIG. 18, the positive cell rate of the comparative example is represented by a bar graph with hatching, and the positive cell rate by the method of the other example of this Modification 1 is represented by a bar graph without hatching.

Regarding the IGH / BCL2 fusion gene of the sample, as shown in FIG. 16, the positive cell rate calculated by the method of this Modification 1 is 63.6%, whereas the positive cell rate calculated by the method of the comparative example is 29.0%. The number of the numerator in the equation for calculating the positive cell rate (63.6%) calculated by the other method of Modification 1 is 1236, and the denominator is 1944. On the other hand, the numerator in the equation for calculating the positive cell rate (29.0%) calculated by the method of the comparative example is 4939, and the denominator is 17058. By setting the region in the manner described in FIG. 17, the numerator for the equation for calculating the positive cell rate used 1236 cells selected from 4939 cells, that is, the selection rate was 25.0%, and the denominator used 1944 cells selected from 17058 cells, that is, the selection rate was 11.4%. As described above, in the other method of the present modification 1, the cell selection rate in the numerator of the equation for calculating the positive cell rate becomes larger than the cell selection rate in the denominator due to the setting of the region described above shown in FIG. 17, and positive cells can be efficiently extracted.

Regarding the IGH / CCND1 fusion gene of this sample, as shown in FIG. 18, the positive cell rate calculated by the other method of this modification 1 is 18.1%, whereas the positive cell rate calculated by the method of the comparative example is 24.1%, and the difference in positive cell rate is less than 7%. Regarding the IGH / MYC fusion gene of this sample, as shown in FIG. 18, the positive cell rate calculated by the other method of this modification 1 is 10.6%, whereas the positive cell rate calculated by the method of the comparative example is 12.3%, and the difference in positive cell rate is less than 2%. In this way, in the other method of the present modification 1, even if the above-described region shown in FIG. 17 is set, the above-described region shown in FIG. 17 is not set for the sample having no IGH translocation, and the positive cell rate can be output with the same accuracy.

As described above, in the other method of Modification 1, the difference between the positive cell rate in the sample having the IGH translocation and the positive cell rate in the sample not having the IGH translocation becomes large, and the positive cell rate can be output as an index for the diagnosis of diffuse large B-cell lymphoma. Therefore, the diagnosis of the subject can be facilitated.

Second Modification

As an image for extracting selection information, a first image which is a bright-field image and a third image which is a fluorescence image of nuclear staining may be used. Then, the selection information also may include information on the cell size other than the cell area extracted from the first image, for example, the diameter or width of the cell. The selection information also may include information on the size of the nucleus other than the nucleus area extracted from the third image, for example, the diameter or width of the nucleus.

Third Modification

As an image for extracting selection information, a first image which is a bright-field image may be used. Other selection information also may be acquired from a signal instead of the image. Specifically, the processing unit 11 may calculate information on the cell size, for example, the cell area based on the first image. The processing unit 11 may use the intensity of fluorescence from the dye-labeled nucleus generated when the cells are irradiated with the excitation light as selection information. Note that the magnitude of the fluorescence intensity from the dye-labeled nucleus indicates the size of the nucleus.

Fourth Modification

A fluorescence image of nuclear staining also may be adopted as the first image for extracting the selection conditions, and the selection information of one or more items may include information about the nucleus calculated by the processing unit 11 based on the fluorescence image of nuclear staining, for example, the area of the nucleus. The processing unit 11 may acquire other selection information from the signal rather than the image, and the processing unit 11 may use the intensity of scattered light (preferably forward scattered light) as the signal for the basis of the selection information.

Note that the forward scattered light is also called low-angle scattered light, and indicates the light scattered forward when the laser light irradiates the cells. The size of the forward scattered light is approximately proportional to the size of the cells.

Fifth Modification

A dark-field image also may be used instead of the bright-field image in the above-described embodiment and all modifications obtained by using the bright-field image for selection information. That is, the dark-field image may be used as the first image, and the processing unit 11 may acquire selection information based on the dark-field image. A dark-field image is an image based on light scattered by cells. Information on the size of cells, for example, information on the cell area can also be obtained by using a dark- field image.

Sixth Modification

In the above embodiment, regarding a plurality of cells contained in the sample 20a, a case where each cell is photographed by the imaging unit 100 and one cell is photographed in each image has been described. However, with respect to one or more of the first to third images, a plurality of cells may be photographed in each image, and information on the plurality of cells may be obtained by analysis of each image. For example, one bright-field image may show a plurality of cells, or one second image may show bright spots of a plurality of cells. Alternatively, a plurality of nuclei may be shown in one nuclei-stained image.

Seventh Modification

For one or more of the first to third images, only a part of the cells also may be captured in each image, and information about a plurality of cells may be obtained by analysis of each image. For example, one nuclear-stained image does not have to show the entire cell, but may show only a part of the cell containing the nucleus.

Eighth Modification

In all the above-described embodiments and modifications, the case of acquiring selection information using at least the first image has been described. However, it is not necessary to acquire selection information. Specifically, whether a cell corresponds to a test cell also may be determined by inputting pixel data extracted from an image into a classification algorithm having a neural network (preferably a deep learning algorithm).

The image is data, and each pixel of the image has brightness information and hue information. In this case, pixel data, for example, information on the magnitude of brightness (information on brightness), information on hue, and the like is input directly to a classification algorithm having a neural network, and whether the cell information corresponds to a test cell is determined by the classification algorithm.

Specifically, first, a large number of images of test cells and images of non-test cells are prepared, and a classification algorithm having a neural network is trained using the large number of images. The trained classification algorithm will be able to determine with considerable accuracy whether a cell is a test cell. Therefore, a threshold value (for example, 80%) can be set, and only cells determined by the classification algorithm to be test cells with a probability of the threshold value or higher can be extracted as test cells.

Modified Example of Calculation of Positive Cell Rate

In the above embodiment, the positive cell rate was calculated using the above equation (1). However, other formulas may be used to calculate the positive cell rate. Hereinafter, a modified example for calculating the positive cell rate using a method different from equation (1) will be described.

First Modification

The positive cell rate may be calculated using the following equation (2).

$$\text{Positive cell rate} = \text{number of positive cells} / (\text{number of tested cells} - \text{number of indeterminate cells}) \ldots (2)$$

In the calculation of the positive cell rate using equation (2), as in the calculation of the positive cell rate using equation (1), the indeterminate cells corresponding to the unclear fluorescence image are excluded, and the positive cell rate is calculated only by the number of negative cells and the number of positive cells. In this way it is possible to output more accurate information on the number of positive cells.

Second Modification

In the above embodiment and the first modification, the positive cell rate was calculated in consideration of indeterminate cells, but the positive cell rate also may be calculated using the following equation (3) without considering indeterminate cells...

$$\text{Positive cell rate} = \text{number of positive cells} / \text{number of test cells} \dots (3)$$

Third Modification

In the above embodiment and each modification, only the primary value in one or more variables of the number of positive cells, the number of tested cells, and the number of indeterminate cells appeared in the equation of positive cells. However, the formula for the positive cell rate may include a function other than the first order with one or more of the number of positive cells, the number of tested cells, and the number of indeterminate cells as variables. For example, the equation for positive cell rate may include the square of one or more variables among the number of positive cells, the number of test cells, and the number of indeterminate cells, and in one specific example, the positive cell rate also may be calculated by dividing the square of the number of positive cells by the number of test cells.

In this way even when functions other than the first order (for example, a higher order function of the second order or higher, exponential function, logarithm) with one or more of the number of positive cells, the number of test cells, and the number of indeterminate cells as variables is included in the equation of the positive cell rate, it corresponds to the case where information on the ratio of cells in which gene fusion occurs is calculated.

Fourth Modification

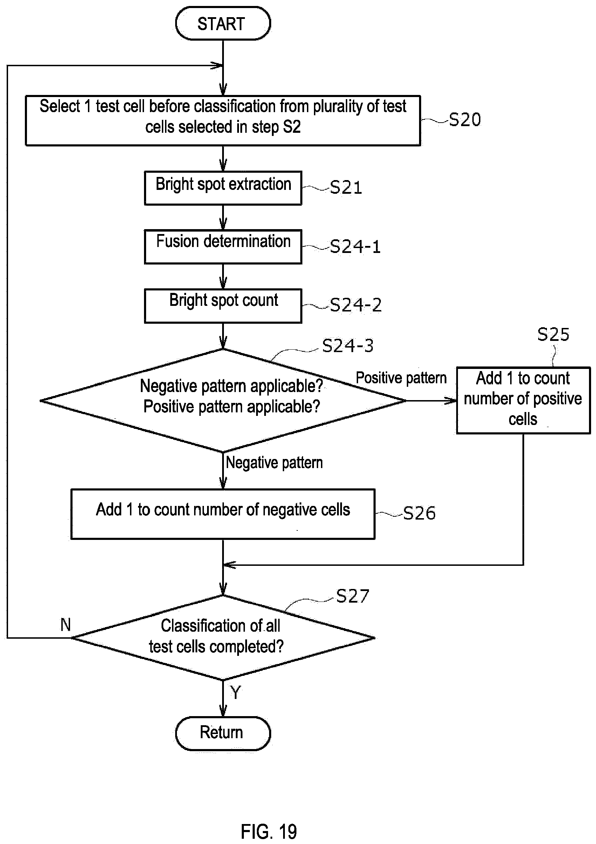

In the positive cell rate of the above embodiment and each modification, the number of positive cells also may be replaced with (number of test cells-number of negative cells) to calculate the positive cell rate. FIG. 19 is a flowchart showing a modified example of the image processing of the processing unit 11 in step S3 of FIG. 10. The flowchart shown in FIG. 19 differs only in that steps S22 and S23 are omitted in comparison with the flowchart of FIG. 12 showing the procedure of image processing of the processing unit 11 in step S3 of the above embodiment.

As shown in FIG. 19, in step S3 of FIG. 10 each cell contained in the plurality of test cells always may be classified into either the number of positive cells or negative cells. In this case, since the number of indeterminate cells does not exist, the positive cell rate is calculated by replacing the positive cell number with (test cell number-negative cell number) in the positive cell rate of the above embodiment and each modification to make an accurate determined. Even when the number of indeterminate cells exists, the positive cell rate may be calculated by replacing the positive cell number with (test cell number-negative cell number) in the positive cell rate of the above embodiment and each modification.

Other Modifications

In the above embodiment, no further treatment was performed on the plurality of cells classified as non-test cells in step S14 shown in FIG. 11. However, further treatment may be performed on a plurality of cells classified as non-test cells in step S14.

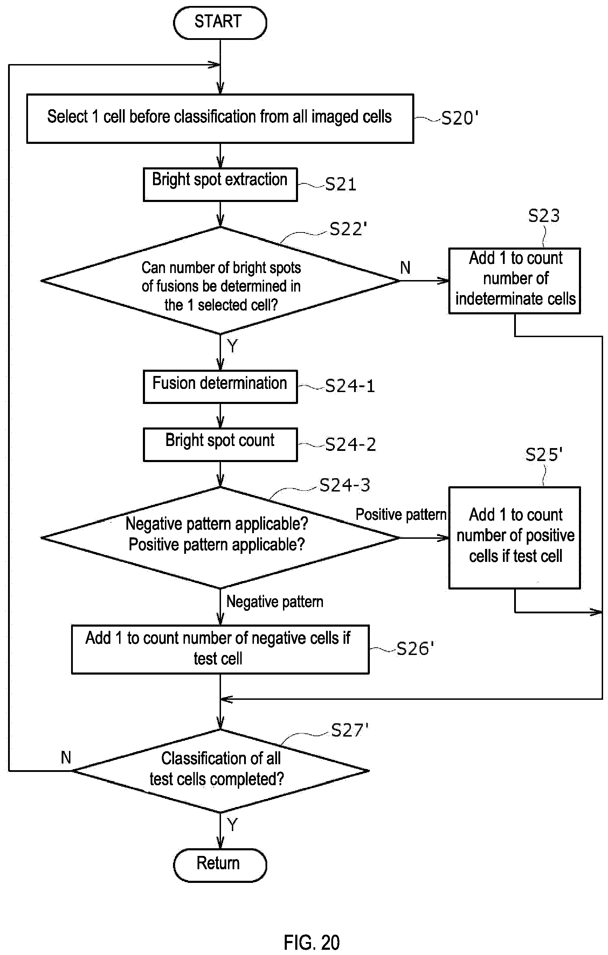

More specifically, as shown in FIG. 20, in step S20', the processing unit 11 selects one cell from all the captured cells and acquires the images (both the first second image and the second second image) are associated with the selected cell from the storage unit 12. In the processes of step S21, step S22', and steps 23 to S24-3, the only difference is the test cell of step S22' was changed to 1 cell in comparison with steps S21 to S24-3 of the above embodiment. Since the processes of step S21, step S22', and steps 23 to S24-3 are basically the same as the processes of steps S21 to S24-3 of the above embodiment, the description thereof will be omitted. In step S25', if the cell ID of the cell is labeled as a test target, the processing unit 11 associates the cell ID with a label indicating that it is a positive cell and stores it in the storage unit 12, and at the same time, the count number of positive cells is increased by 1, and the number of updated positive cells is stored in the storage unit 12. If not labeled as being a test target, the positive cell count is unchanged. In step S26', if the cell ID of the cell is labeled as a test target, the processing unit 11 associates the cell ID with a label indicating that it is a negative cell and stores it in the storage unit 12, and at the same time the count number of negative cells is increased by 1, and the number of updated negative cells is stored in the storage unit 12. If not labeled as being a test target, the positive cell count is unchanged. In step S27', the processing unit 11 determines whether the processing for all the imaged cells has been completed. If the determination in step S27'is affirmative, the processing unit 11 returns the processing to step S4 in FIG. 10, and if the determination is negative, the processing unit returns the processing to step S20'.

The above-described embodiment and modification has been described in terms of the case in which fusion determination of fluorescence bright spots is performed. However, the cells included in the image also may be classified into positive cells and negative cells based on the number of fluorescent bright spots without performing the fusion determination of the fluorescent bright spots. Since the cell classification method, that is, the index used for classification differs depending on the cell to be analyzed, the target site, the measurement item, the fluorescent label, and the like, it is necessary to use an appropriate classification index according to the analysis target.

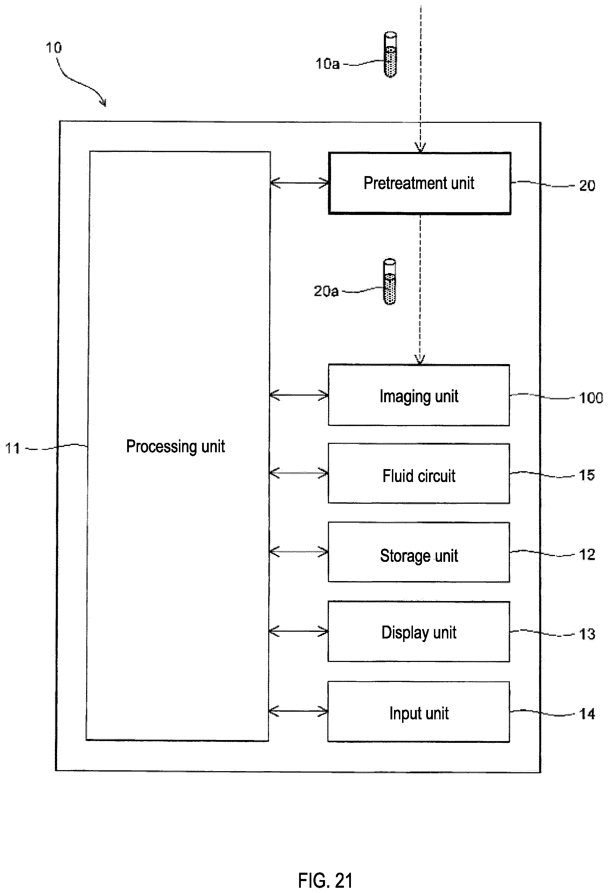

As shown in FIG. 21, the fluorescence image analyzer 10 may include a pretreatment unit 20 in the apparatus. The processing unit 11 is connected to the preprocessing unit 20 and is configured to be able to control the preprocessing unit 20. When the sample 10a collected from the subject and subjected to processing such as centrifugation is set, the pretreatment unit 20 performs pretreatment on the sample 10a and prepares the sample 20a containing cells having a target site that is fluorescently labeled. Note that the other configurations are the same as those shown in FIG. 1 As in the embodiment illustrated in FIG. 21, when the fluorescence image analyzer 10 includes the pretreatment unit 20, the operator simply sets the sample 10a in the device, and the pretreatment is automatically performed and prepared, and the sample 20a is then automatically analyzed.

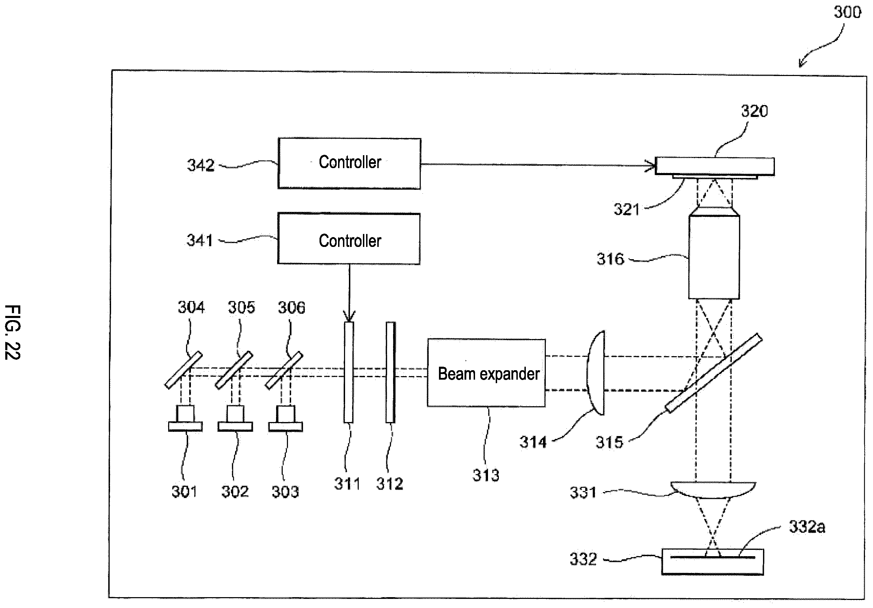

As shown in FIG. 22, an imaging unit 300 including a fluorescence microscope may be provided instead of the imaging unit 100 exemplified in FIG. 1. The imaging unit 300 includes light sources 301 to 303, mirrors 304, dichroic mirrors 305 and 306, shutter 311, 1/4 wavelength plate 312, beam expander 313, condenser lens 314, dichroic mirror 315, an objective lens 316, a stage 320, a condenser lens 331, an image capture unit 332, and controllers 341 and 342.

The stage 320 is a support base on which the slide glass 321 is installed, and is driven by the controller 342. The sample 20a prepared by the pretreatment unit 20 is placed on the slide glass 321 installed on the stage 320. That is, in the imaging unit 300, the fluorescently labeled cells on the slide glass 321 are imaged by a fluorescence microscope provided with the image capture unit 332, and a fluorescence image is acquired.

The light sources 301 to 303 are the same as the light sources 121 to 123 shown in FIG. 1, respectively. The mirror 304 reflects the light from the light source 301. The dichroic mirror 305 transmits the light from the light source 301 and reflects the light from the light source 302. The dichroic mirror 306 transmits the light from the light sources 301 and 302 and reflects the light from the light source 303. The optical axes of the light from the light sources 301 to 303 are aligned with each other by the mirror 304 and the dichroic mirrors 305 and 306.

The shutter 311 is driven by the controller 341 and switches between a state in which the light emitted from the light sources 301 to 303 passes through and a state in which the light emitted from the light sources 301 to 303 is blocked. In this way the irradiation time of light on the sample 20a is adjusted. The quarter wave plate 312 converts the linearly polarized light emitted from the light sources 301 to 303 into circularly polarized light. The fluorescent dye bound to the nucleic acid probe reacts to light in a predetermined polarization direction. Therefore, by converting the excitation light emitted from the light sources 301 to 303 into circular polarization, the polarization direction of the excitation light tends to match the polarization direction with which the fluorescent dye reacts. In this way it possible to efficiently excite the fluorescent dye.

The beam expander 313 expands the irradiation area of light on the slide glass 321. The condenser lens 314 collects light so that the slide glass 321 is irradiated with parallel light from the objective lens 316. The dichroic mirror 315 reflects the light emitted from the light sources 301 to 303 and transmits the fluorescence generated from the sample 20a. The objective lens 316 guides the light reflected by the dichroic mirror 315 to the slide glass 321.

The fluorescence generated from the sample 20a passes through the objective lens 316 and passes through the dichroic mirror 315. The condenser lens 331 collects the fluorescence transmitted through the dichroic mirror 315 and guides it to the image capture surface 332a of the image capture unit 332. The image capture unit 332 takes an image of the fluorescence irradiated on the image capture surface 332a and generates a fluorescence image. The image capture unit 332 is composed of, for example, a CCD or the like.

The controllers 341 and 342 and the image capture unit 332 are connected to the above-mentioned processing unit 11. The processing unit 11 controls the controllers 341 and 342 and the image capture unit 332, and receives the fluorescence image captured by the image capture unit 332. In the fluorescence image captured by the image capture unit 332, the cells may be in close contact with each other, unlike the case where the flow cell 110 is used. Therefore, the processing unit 11 performs a process of dividing the acquired fluorescence image into nuclei of cells, a process of setting a region corresponding to the nucleus of one cell in the fluorescence image, and the like.

Also in the fluorescence image analyzer provided with the imaging unit 300, the test cells are classified based on the acquired fluorescence image, the occurrence of gene fusion is determined for the test cells, and the positive cell rate is calculated. In this way it possible to output information on the proportion of positive cells without isolating specific types of cells. Therefore, the diagnosis of the subject can be facilitated.

Note that when carrying out the fluorescence image analysis method of the present invention, a fluorescence image analysis program that causes a computer to perform the following processes (a) to (e) also may be used.

(a) A process of acquiring a first image in which at least a part of a region of a cell is included in an image capture target and a second image in which the fluorescence generated from a fluorescent dye labeled with a target site of the cell in the first image is included in the image capture target, performed for a plurality of cells in a sample that are included in the sample and have target site on the chromosome labeled with a fluorescent dye.

(b) A process of selecting a plurality of test cells to be tested from a plurality of cells based on at least the first image.

(c) A process of extracting bright spots due to fluorescence generated from a fluorescent dye from at least a plurality of test cells based on a second image.

(d) A process for identifying cells with chromosomal abnormalities and/or cells without chromosomal abnormalities based on the extracted bright spots.

(e) A process of generating information about the proportion of cells with chromosomal abnormalities based on the number of cells tested, the number of cells with chromosomal abnormalities and/or the number of cells without chromosomal abnormalities.

The gene fusion that can be inspected by the method of the present disclosure is not limited to the gene fusion used in the description in the above embodiment, insofar as any other gene fusion also may be used. For example, t (14; 20), t (8; 14), or IGH split (14q32) may be detected by the method of the present disclosure. t (14; 20), t (8; 14), and IGH split (14q32) are translocations of a gene that cause multiple myeloma, similar to t (11; 14), t (4; 14), and t (14; 16). In addition, t (3; 14), MYC split (8q24), BCL2 split (18q21), or BCL6 split (3q27) may be detected by the method of the present disclosure. t (3; 14), MYC split (8q24), BCL2 split (18q21), and BCL6 split (3q27) are translocations of a gene that cause malignant lymphoma similar to t (11; 14), t (14; 18), and t (8; 14). I

The method of the present disclosure also may detect a chromosomal abnormality other than gene fusion due to a chromosomal translocation, and may detect a chromosomal inversion, a gene deletion, a gene amplification, or the like as a chromosomal abnormality. For example, del (17p), del (13q), or del (1p) may be detected as a gene deletion, and for example, gain (1q21) may be detected as a gene amplification. Here, del means deletion, gain means amplification, and the number in parentheses means the locus of each gene. When detecting a gene deletion, the processing unit 11 omits the processing of step S24-1 in FIG. 12, and in step S24-3 the number of bright spots counted in step S24-2 is compared with the number of the gene in the negative cells, and if both are the same, the process proceeds to step S26, whereas when the number of bright spots counted is less than the number of the gene in the negative cells, the process proceeds to step S25. When detecting a gene amplification, the processing unit 11 omits the processing of step S24-1 in FIG. 12, and in step S24-3 the number of bright spots counted in step S24-2 is compared with the number of the gene in the negative cells, and if both are the same, the process proceeds to step S26, whereas when the number of bright spots counted is greater than the number of the gene in the negative cells, the process proceeds to step S25.

The diseases that can be inspected by the method of the present disclosure are not limited to the diseases mentioned in the above-described embodiments and modifications.

**Claims**

1. A fluorescence image analysis method comprising:

labeling target sites on chromosomes with first fluorescent dye in a plurality of cells contained in a sample;
capturing, for the plurality of the cells in the sample, first images containing at least parts of the cells, and second images containing fluorescence generated from the first fluorescent dye labeled at the target sites of the cells in the first images;
selecting a plurality of test cells to be tested and having specific morphological characteristics from the plurality of cells based on at least the first images;
extracting bright spots due to the fluorescence generated from the first fluorescent dye from at least a plurality of the test cells based on the second images;
identifying cells with chromosomal abnormalities and/or cells without chromosomal abnormalities based on the extracted bright spots; and
generating information related to a ratio of the cells with chromosomal abnormality to the test cells based on a number of the cells with chromosomal abnormality and/or a number of the cells without chromosomal abnormality.

2. The fluorescence image analysis method according to claim 1, wherein
in the extracting the bright spots, cells from which the bright spots are extracted are the test cells selected in the selecting the plurality of test cells.

3. The fluorescence image analysis method according to claim 1 or 2, wherein
the selecting the plurality of test cells comprises

acquiring selection information from the first image; and
selecting the plurality of test cells based on the acquired selection information.

**4.** The fluorescence image analysis method according to claim 3, wherein
the selection information comprises at least one of a value relating to a size of a cell, a value relating to a size of a nucleus of a cell, and a value relating to uneven distribution of a nucleus within a cell.

**5.** The fluorescence image analysis method according to claim 3, wherein
the selection information comprises a value relating to a size of a cell and a value relating to uneven distribution of a nucleus within a cell.

**6.** The fluorescence image analysis method according to claim 3, wherein
the selection information comprises a value relating to a size of a cell and a value relating to a size of a nucleus within a cell.

**7.** The fluorescence image analysis method according to any one of claims 1 to 6, wherein
the first image comprises a bright-field image obtained by detecting light transmitted through a cell.

**8.** The fluorescence image analysis method according to any one of claims 1 to 7, wherein
the first image contains a cytoplasm and a nucleus of a cell.

**9.** The fluorescence image analysis method according to any one of claims 1 to 8, further comprising

labeling nuclei of the plurality of cells with second fluorescent dye having an output wavelength different from that of the first fluorescent dye before capturing the first images and the second images; and wherein
the capturing further comprises capturing a third image containing fluorescence generated from the second fluorescent dye labeled with the nuclei of the cells in the first images; and
the selecting the plurality of test cells comprises selecting the plurality of the test cells based on at least the first images and the third images.

**10.** The fluorescence image analysis method according to claim 9, wherein
the selecting the plurality of test cells comprises acquiring selection information from the first images and the third images, and selecting the plurality of test cells based on the acquired selection information.

**11.** The fluorescence image analysis method according to claim 10, wherein

the first image comprises a bright-field image obtained by detecting light transmitted through a cell; and
the selection information comprises a value relating to an area of a cell obtained from the first image, and a value relating to uneven distribution of a nucleus within a cell obtained from the first image and the third image.

**12.** The fluorescence image analysis method according to claim 10, wherein

the first image comprises a bright-field image obtained by detecting light transmitted through a cell; and
the selection information comprises a value relating to an area of a cell obtained from the first image and a value relating to an area of a nucleus obtained from the third image.

**13.** The fluorescence image analysis method according to any one of claims 1 to 12, wherein
the chromosomal abnormality comprises at least one of chromosomal translocation, chromosomal inversion, gene deletion, and gene amplification.

**14.** The fluorescence image analysis method according to any one of claims 1 to 13, wherein
the chromosomal abnormality comprises a translocation of a gene caused by multiple myeloma or malignant lymphoma.

**15.** The fluorescence image analysis method according to claim 14, wherein
a fusion gene generated by the translocation comprises an IGH/FGFR3 fusion gene, an IGH/CCND1 fusion gene, an IGH/MAF fusion gene, an IGH/BCL2 fusion gene, or an IGH/MYC fusion gene.

**16.** The fluorescence image analysis method according to any one of claims 1 to 15, wherein
the generating information related to the ratio comprising generating the information related to the ratio without including, in a number of the test cells, a number of test cells that could not be identified based on the bright spots.

**17.** The fluorescence image analysis method according to any one of claims 1 to 16, wherein
the generating information related to the ratio comprising generating the ratio of the cells with chromosomal abnormality to the test cells by:
dividing a number of the cells with chromosomal abnormality by a sum of a number of cells selected as the test cells and having chromosomal abnormality and a number of cells selected as the test cells and without having chromosomal abnormalities.

**18.** The fluorescence image analysis method according to any one of claims 1 to 17, wherein
the first images and the second images are captured in a state in which the sample is flowing.

**19.** The fluorescence image analysis according to any one of claims 1 to 18, wherein
the capturing the first images and the second images comprises capturing the first image and the second image for each of the plurality of cells.

**20.** A fluorescence image analyzer comprising:

an imaging unit for capturing, for a plurality of cells in which target sites on chromosomes are labeled with fluorescent dye, and the cells being contained in a sample, first images containing at least parts of the cells in the sample, and capturing second images containing fluorescence generated from the fluorescent dye labeling the target sites of the cells in the first images; and
a processing unit;
wherein the processing unit executes

selecting a plurality of test cells to be tested and having specific morphological characteristics from the plurality of cells based on at least the first images;
extracting bright spots due to the fluorescence generated from the fluorescent dye from at least a plurality of the test cells based on the second images;
identifying cells with chromosomal abnormalities and/or cells without chromosomal abnormalities based on the extracted bright spots, and
generating information related to a ratio of the cells with chromosomal abnormality to the test cells based on a number of the cells with chromosomal abnormality and/or a number of cells without chromosomal abnormality.

**21.** A fluorescence image analysis computer-executable program for executing

obtaining, for a plurality of cells in which target sites on chromosomes are labeled with fluorescent dye, and the cells being contained in a sample, first images containing at least parts of the cells in the sample and second images containing fluorescence generated from the fluorescent dye labeling the target sites of the cells in the first image;
selecting a plurality of test cells to be tested and having specific morphological characteristics from the plurality of cells based on at least the first images;
extracting bright spots due to the fluorescence generated from the fluorescent dye from at least a plurality of the test cells based on the second images;
identifying cells with chromosomal abnormality and/or cells without chromosomal abnormality based on the extracted bright spots; and
generating information related to a ratio of cells with chromosomal abnormality to the test cells based on a number of cells with chromosomal abnormality and/or a number of cells without chromosomal abnormality.

FIG. 1

| 1st Image | First 2nd Image | Second 2nd Image | Composite Image | 3rd Image |
|---|---|---|---|---|

FIG. 2

FIG. 3

FIG. 4

Multiple myeloma cell

FIG. 5A (a)    FIG. 5A (b)    FIG. 5A (c)
Nuclear image  Bright-field image  Composite image

Non-multiple myeloma cell

FIG. 5B (a)    FIG. 5B (b)    FIG. 5B (c)
Nuclear image  Bright-field image  Composite image

FIG. 6A

3rd image

Frequency

Nuclear region

Threshold
Value

Brightness

FIG. 6B

1st Second image

Frequency

Bright spot region

Threshold
Value

Brightness

FIG. 6C

2nd Second image

Frequency

Bright spot region

Threshold
Value

Brightness

FIG. 7A  Negative pattern

1st Second image          2nd Second image          Composite image

Nucleus
Red
Green

G2R2F0

FIG. 7B  Positive pattern 1

1st Second image          2nd Second image          Composite image

Nucleus
Red
Green
Yellow

G2R3F1

FIG. 7C  Positive pattern 2

1st Second image          2nd Second image          Composite image

Nucleus
Red
Green
Yellow

G3R3F2

FIG. 7D  Positive pattern 3

1st Second image          2nd Second image          Composite image

Nucleus
Green
Red
Yellow

G2R2F1

FIG. 8A
Capture image

FIG. 8B
Noise removal

FIG. 8C
Binarization

FIG. 8D
Center of gravity coordinate Determination

FIG. 8E
Fusion determination

1st Second image

2nd Second image

EP 4 137 795 A1

FIG. 9

START

Capture bright-field image, two types FISH stained image, and nuclear-stained image of a plurality of cells in sample ~S1

Select test cells from plurality of cells ~S2

Classify plurality of test cells as indeterminate cell, positive cell, or negative cell ~S3

Calculate positive cell rate ~S4

END

FIG. 10

START

Select one pre-classification cell from a plurality of cells in the sample; acquire image — S11

Calculate cell area and nuclear uneven distribution value og one selected pre-classified cell — S12

S13
Cell area exceeds threshold value? Nuclear uneven distribution value exceeds threshold value?

S14
N → Classify as non-test cell

Y

Classify as test cell — S15

S16
All cells in sample have been classified?

N

Y

Return

FIG. 11

START

Select 1 test cell before classification from plurality of test cells selected in step S2 ～S20

Extract bright spots ～S21

S22
Can number of bright spots of fusions be determined in selected test cell? —N→ Add 1 to count number of indeterminate cells S23

Y

Fusion determination ～S24-1

Bright spot count ～S24-2

S24-3
Negative pattern applicable? Positive pattern applicable? —Positive pattern→ Add 1 to count number of positive cells S25

Negative pattern

Add 1 to count number of negative cells ～S26

S27
Classification of all test cells completed? —N→

Y

Return

FIG. 12

IGH/FGFR3 Positive

FIG. 13

Translocation negative

FIG. 14

Diffuse large B-cell lymphoma (lymph node)

FIG. 15

FIG. 16

Diffuse large B-cell lymphoma (lymph node)

FIG. 17

FIG. 18

START

Select 1 test cell before classification from plurality of test
cells selected in step S2 ~S20

Bright spot extraction ~S21

Fusion determination ~S24-1

Bright spot count ~S24-2

S24-3
Negative pattern applicable?          Positive pattern    S25
Positive pattern applicable?  ——————————————→  Add 1 to count
                                                number of positive
                                                cells

Negative pattern

Add 1 to count number of negative cells ~S26

S27
N ←——  Classification of all
        test cells completed?

Y

Return

FIG. 19

START

Select 1 cell before classification from all imaged cells ⟶ S20'

Bright spot extraction ⟶ S21

S22'

Can number of bright spots of fusions be determined in the 1 selected cell? —— N ⟶ Add 1 to count number of indeterminate cells ⟶ S23

Y

Fusion determination ⟶ S24-1

Bright spot count ⟶ S24-2

S24-3

Negative pattern applicable? Positive pattern applicable? —— Positive pattern ⟶ Add 1 to count number of positive cells if test cell ⟶ S25'

Negative pattern

Add 1 to count number of negative cells if test cell ⟶ S26'

S27'

N —— Classification of all test cells completed?

Y

Return

FIG. 20

FIG. 21

FIG. 22

Europäisches
Patentamt

European
Patent Office

Office européen
des brevets

# EUROPEAN SEARCH REPORT

Application Number

EP 22 19 0967

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/232019 A1 (ERBER WENDY NAOMI [AU] ET AL) 23 July 2020 (2020-07-23) * abstract; figures 1-16; tables 1, 10 * * paragraphs [0005], [0010], [0011], [0031], [0037], [0041], [0042], [0044], [0048], [0063], [0075] - paragraphs [0203], [0204], [0205], [0207] * | 1-21 | INV. G01N15/14 G01N21/64 G06T7/00 G01N15/10 |
| X | JP 2019 086524 A (SYSMEX CORP) 6 June 2019 (2019-06-06) * abstract; figures 1-2 * * paragraphs [0006], [0013], [0022], [0026], [0034], [0047] * | 1-21 | |
| X | EP 3 388 820 A1 (SYSMEX CORP) 17 October 2018 (2018-10-17) * abstract; figures 1-15 * * paragraphs [0037] - [0069] * | 1-21 | |
| X | MA EDMOND S. K. ET AL: "Target fluorescence in-situ hybridization (Target FISH) for plasma cell enrichment in myeloma", MOLECULAR CYTOGENETICS, [Online] vol. 9, no. 1, 16 December 2016 (2016-12-16), XP093009938, DOI: 10.1186/s13039-016-0263-7 Retrieved from the Internet: URL:http://link.springer.com/content/pdf/10.1186/s13039-016-0263-7.pdf> [retrieved on 2022-12-20] * abstract; figures 1, 2 * * whole document, in particular page 7 * | 1-16, 19-21 | TECHNICAL FIELDS SEARCHED (IPC) G01N G06T |

The present search report has been drawn up for all claims

| Place of search | Date of completion of the search | Examiner |
|---|---|---|
| The Hague | 23 December 2022 | Zarowna-Dabrowska, A |

EPO FORM 1503 03.82 (P04C01)

## ANNEX TO THE EUROPEAN SEARCH REPORT
## ON EUROPEAN PATENT APPLICATION NO.

EP 22 19 0967

This annex lists the patent family members relating to the patent documents cited in the above-mentioned European search report.
The members are as contained in the European Patent Office EDP file on
The European Patent Office is in no way liable for these particulars which are merely given for the purpose of information.

23-12-2022

| Patent document cited in search report | | Publication date | Patent family member(s) | | Publication date |
|---|---|---|---|---|---|
| US 2020232019 | A1 | 23-07-2020 | AU | 2018355889 A1 | 16-04-2020 |
| | | | CN | 111448324 A | 24-07-2020 |
| | | | EP | 3701045 A1 | 02-09-2020 |
| | | | JP | 2021501885 A | 21-01-2021 |
| | | | US | 2020232019 A1 | 23-07-2020 |
| | | | WO | 2019079851 A1 | 02-05-2019 |
| JP 2019086524 | A | 06-06-2019 | JP | 6948354 B2 | 13-10-2021 |
| | | | JP | 2019086524 A | 06-06-2019 |
| EP 3388820 | A1 | 17-10-2018 | CN | 108732144 A | 02-11-2018 |
| | | | EP | 3388820 A1 | 17-10-2018 |
| | | | JP | 6959755 B2 | 05-11-2021 |
| | | | JP | 2018174828 A | 15-11-2018 |
| | | | US | 2018299382 A1 | 18-10-2018 |
| | | | US | 2021302315 A1 | 30-09-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021133166 A
- JP 2017215311 A

- US 20200271585 A